# EUROPÄISCHE PATENTSCHRIFT

(11) **EP 3 830 069 B1**
(45) Veröffentlichungstag und Bekanntmachung des Hinweises auf die Patenterteilung: **07.09.2022**
(21) Anmeldenummer: 19742221.5
(22) Anmeldetag: 24.07.2019
(51) Int. Cl.: C07C 209/68, C07C 211/46, C12P 7/40

(54) **VERFAHREN ZUR HERSTELLUNG VON ANILIN ODER EINES ANILIN-FOLGEPRODUKTS**
METHOD FOR THE PREPARATION OF AMINOBENZOIC ACID OR AN AMINOBENZOIC ACID DERIVATIVE
PROCÉDÉ DE FABRICATION D'ANILINE OU D'UN PRODUIT DÉRIVÉ D'ANILINE

(30) Priorität: 27.07.2018 EP 18186072
(43) Veröffentlichungstag der Anmeldung: 09.06.2021
(73) Patentinhaber: Covestro Intellectual Property GmbH & Co. KG, 51373 Leverkusen (DE)
(72) Erfinder: LANGANKE, Jens, 53894 Mechernich (DE); BEGGEL, Franz, 50935 Köln (DE); JÄGER, Gernot, 50733 Köln (DE); KLOECKNER, Wolf, 51065 Köln (DE); MICHELE, Volker, 51065 Köln (DE); VÖSSING, Thomas, 40593 Düsseldorf (DE)
(74) Vertreter: Levpat
(86) Internationale Anmeldenummer: PCT/EP2019/069859
(87) Internationale Veröffentlichungsnummer: WO 2020/020919

(56) Entgegenhaltungen:
- WO-A1-2018/002088
- JP-A- 2016 222 575
- US-A- 5 145 958

## Beschreibung

Die vorliegende Erfindung betrifft ein Verfahren zur Herstellung von Anilin oder eines Anilinfolgeprodukts, bei dem eine Lösung von Aminobenzoesäure in Anilin mit einem auf die Gesamtmasse aus Aminobenzoesäure und Anilin bezogenen Massenanteil an Anilin in der Lösung im Bereich von 20 % bis 85 % einer thermischen Decarboxylierung bei einer Temperatur im Bereich von 165 °C bis 500 °C ohne Anwesenheit eines systemfremden Katalysators unterworfen wird, sodass die Aminobenzoesäure zu Anilin umgesetzt wird. Das erhaltene Anilin kann zu Folgeprodukten wie beispielsweise den Di- und Polyaminen der Diphenylmethanreihe umgesetzt werden.

Die Herstellung von Anilin durch Decarboxylierung von Aminobenzoesäure ist grundsätzlich im Stand der Technik bekannt; siehe beispielsweise Per Wiklund et al., Current Organic Synthesis, 2006, 3, 379 - 402. In Stevens et al., Canadian Journal of Chemistry, 1952, 30 (7), 529 - 540, wird berichtet, dass eine wässrige Lösung von ortho-Aminobenzoesäure in Gegenwart von 0,75 N Schwefelsäure in 6 Stunden bei 100 °C mit 56%iger Ausbeute zu Anilin decarboxyliert werden konnte. In MacMaster and Shriner, J. Am. Chem. Soc., 1923, 45 (3), 751 - 753 war zuvor berichtet worden, dass unter ähnlichen Bedingungen (in siedendem Wasser) aber in Abwesenheit von Säure ortho-Aminobenzoesäure in 7 Stunden mit nur 27%iger Ausbeute zu Anilin decarboxyliert wurde.

Auch in der jüngeren Patentliteratur finden sich hierzu Veröffentlichungen; siehe hierzu insbesondere die internationalen Veröffentlichungen WO 2015/124686 A1 und WO 2015/124687 A1:
In WO 2015/124686 A1 wird die Decarboxylierung *des Anions* der Anthranilsäure, ortho-Aminobenzoat, insbesondere in Form des Ammoniumsalzes Ammonium-ortho-Aminobenzoat, in Gegenwart eines Katalysators oder ohne Katalysator in wässrigem Medium beschrieben, wobei das ortho-Aminobenzoat vorzugsweise fermentativ gewonnen wird. Der in WO 2015/124686 A1 beschriebene Ansatz, eine fermentativ bereitgestellte wässrige Lösung von ortho-Aminobenzoat, gegebenenfalls nach Abtrennung von Biomasse, direkt zu decarboxylieren, ist durchaus nicht per se unattraktiv. Allerdings erfordert das in WO 2015/124686 A1 beschriebene Verfahren die Extraktion von in der Decarboxylierung gebildetem Anilin mit einem systemfremden organischen Lösungsmittel (ein Alkohol, Phenol, Amid, Ether oder aromatischer Kohlenwasserstoff; insbesondere wird 1-Dodecanol als geeignetes Lösungsmittel hervorgehoben), was unvermeidbar mit zusätzlichen Kosten und zusätzlichem Reinigungsaufwand (Trennung Anilin von 1-Dodecanol) verbunden ist. Darüber hinaus muss die gesamte Fermentationsbrühe, die einen signifikanten Anteil an Wasser enthält (typischerweise > 80 Massen-%) aufgeheizt werden, um die Decarboxylierung zu Anilin zu initiieren, was unvermeidbar mit hohen Energiekosten verbunden ist.

In WO 2015/124687 A1 wird die katalytische Decarboxylierung von Anthranilsäure (ortho-Aminobenzoesäure) unter Einwirkung von Zeolith-Katalysatoren in 1-Decanol als Lösungsmittel beschrieben. WO 2015/124687 A1 beschreibt die Durchführung der Decarboxylierung u. a. in Wasser oder in einem systemfremden organischen Lösungsmittel, insbesondere 1-Dodecanol, gegebenenfalls im Gemisch mit Anilin (vgl. Seite 18, Zeilen 28 und 29). Die zuvor geschilderten Nachteile des Einsatzes systemfremder organischer Lösungsmittel sind daher auch für diese Ausführungsformen der Decarboxylierung relevant. Darüber hinaus beschreibt diese Schrift auch die Möglichkeit der Durchführung der Decarboxylierung in Anilin (ohne 1-Dodecanol; siehe die Abbildungen 35 und 37 bis 38 und die zugehörigen Textstellen), gegebenenfalls in Gegenwart von 10 Massen-% Wasser (siehe die Abbildung 36 und die zugehörigen Textstellen). Die Beschreibung dieser Verfahrensvariante geht über das Aufzeigen der grundsätzlichen Möglichkeit einer solchen Decarboxylierung von Aminobenzoesäure aus unterschiedlichen Quellen in Anilin nicht hinaus. Die Decarboxylierung in Anilin ohne weiteren Katalysator bei einer Temperatur von 160 °C wurde als erfolglos beschrieben (nur vernachlässigbare Umsätze).

Beide Anmeldungen beschreiben ferner die weitere Umsetzung des so hergestellten Anilins zu Anilinfolgeprodukten wie den Di- und Polyaminen der Diphenylmethanreihe und den korrespondierenden Isocyanaten.

Die japanische Patentanmeldung JP 2016/222575 beschreibt ein Verfahren zur Gewinnung eines Anti-Alterungsmittels oder Vulkanisierungsbeschleunigers durch Hydrolyse von Indigo, der seinerseits durch Extraktion von Pflanzen gewonnen wird, unter Gewinnung von 2-Aminobenzoesäure, Decarboxylierung der 2-Aminobenzoesäure zu Anilin und anschließende weitere Umsetzung des so gebildeten Anilins. Der Decarboxylierungsschritt kann in Gegenwart einer Säure wie Essigsäure oder Schwefelsäure oder auch ohne solche Säuren durchgeführt werden. Im Beispiel erfolgt die Decarboxylierung ohne Zusatz von Säuren oder anderen Verbindungen in Stickstoffatmosphäre bei 190 °C und Umgebungsdruck.

Die internationale Patentanmeldung WO 2017/085170 A1 beschreibt eine bevorzugte Ausgestaltung der Isolierung von fermentativ gewonnener Aminobenzoesäure aus der erhaltenen Fermentationsbrühe durch Kristallisation. Ebenfalls beschrieben wird die Möglichkeit, die so isolierte Aminobenzoesäure durch Decarboxylierung in Anilin zu überführen. Eine autokatalytische Wirkung des gebildeten Anilins in der Decarboxylierung, die den Verzicht auf einen zusätzlichen Katalysator in einem großtechnischen Verfahren ermöglichen könnte, ist dieser Schrift nicht zu entnehmen.

Die internationale Patentanmeldung WO 2018/002088 A1 beschreibt die Decarboxylierung von Aminobenzoesäure zu Anilin in Gegenwart eines Katalysators, insbesondere eines Zeolithen, wobei ein Teil des gebildeten Rohanilins zurückgeführt und als Lösungsmittel verwendet wird.

Die noch unveröffentlichte internationale Patentanmeldung mit der Anmeldenummer PCT/EP2017/083374 befasst sich mit dem Schritt der Isolierung von Aminobenzoesäure aus einer wässrigen Fermentationslösung unter Anwendung von Adsorptions- und Desorptionsschritten. Eine autokatalytische Wirkung des gebildeten Anilins in der Decarboxylierung, die den Verzicht auf einen zusätzlichen Katalysator in einem großtechnischen Verfahren ermöglichen könnte, ist dieser Schrift nicht zu entnehmen.

Die japanische Patentanmeldung JP 2013-230993 beschreibt die Decarboxylierung von para- und ortho-Aminobenzoesäure in geschmolzenem Zustand oder in wässriger oder in alkoholischer Lösung ohne Katalysator bei Drücken im Bereich von Umgebungsdruck bis 1,0 MPa(ü). Es wird empfohlen, das gebildete Anilin kontinuierlich zu entfernen, weil es ansonsten die Reaktion inhibieren würde. So wird in Vergleichsbeispiel 1 berichtet, dass die Reaktion von para-Aminobenzoesäure in Anilin bei einem Anilingehalt von 89 Massen-% bei 200 °C nach 3 Stunden Reaktionszeit zu 11,1 % Umsatz und 2,9 % Ausbeute ergab.

Das US-amerikanische Patent US 5,145,958 beschreibt ein Verfahren zur Herstellung von 2,4-bzw. 2,6*-Dihalogen-*anilin durch Umsetzung eines Aminobenzoesäureesters mit einem Chlorierungs- oder Bromierungsmittel in einem organischen Lösungsmittel wie insbesondere Chlorbenzol, Dichlorbenzol, Tetrachlorkohlenwasserstoff oder Brombenzol und anschließender Verseifung und Decarboxylierung in Wasser oder in einem Wasser enthaltenden Lösungs- oder Verdünnungsmittel wie insbesondere Toluol, Chlorbenzol, Essigsäure, Salzsäure oder Schwefelsäure. Die Herstellung von (nicht halogensubstituiertem) Anilin wird nicht beschrieben.

Die für die Herstellung von Anilin durch Decarboxylierung erforderliche Ausgangsverbindung Aminobenzoesäure kann (neben Verfahren die letztlich auf der Extraktion von Pflanzen beruhen wie oben im Zusammenhang mit der Diskussion von JP 2016/222575 beschrieben) chemisch oder - im Rahmen der vorliegenden Erfindung bevorzugt - fermentativ hergestellt werden.

Die *chemische* Herstellung von Aminobenzoesäure ist in der Literatur beschrieben. Eine geeignete Syntheseroute (mit Ausbeuten > 98 %) ist beispielsweise die Reaktion von Phthalimid mit Natriumhypochlorit. Phthalimid kann seinerseits aus Phthalsäureanhydrid und Ammoniak gewonnen werden. Der ganze Prozess ist wohlbekannt und wird z. B. in Lorz et al., Phthalic Acid and Derivatives in Ullmann's Encyclopedia of Industrial Chemistry, Band 27, S. 140 - 141, Weinheim, Wiley-VCH, beschrieben. Ein industrieller Prozess ist ebenfalls in der Patentliteratur beschrieben; siehe z. B. DE 29 02 978 A1 und EP 0 004 635 A2.

Die *fermentative* Herstellung von Aminobenzoesäure ist in der Literatur beschrieben. Für die fermentative Herstellung von Aminobenzoesäure sei beispielhaft auf Balderas-Hemandez, V. E. et al., "Metabolic engineering for improving anthranilate synthesis from glucose in Escherichia coli", Microb. Cell. Fact. 2009, 8, 19 (doi: 10.118611475-2859-8-19) verwiesen. Auch in der Patentliteratur finden sich hierzu Veröffentlichungen; siehe beispielsweise die bereits erwähnten Anmeldungen WO 2015/124686 A1 und WO 2015/124687 A1 sowie die darin jeweils zitierte Literatur. Fermentationsprozesse laufen in einer wässrigen Umgebung ab und liefern im Falle der Herstellung von Aminobenzoesäure im Allgemeinen wässrige Lösungen (Fermentationsbrühen) mit einem Massengehalt an Aminobenzoesäure (gegebenenfalls im Form des Anions) im Bereich von 10,0 g/L bis 100 g/L.

Aktuell gibt es keine "biobasierten Verfahren" (im Sinne der vorliegenden Erfindung: Herstellverfahren auf Basis nachwachsender Rohstoffe unter Anwendung fermentativer Prozesse) zur Herstellung von Aminobenzoesäure oder Anilin, welche kommerziell und in großem Produktionsmaßstab durchgeführt werden. Folglich ist - im industriellen Maßstab - auch keine Folgesynthese auf Basis von biobasierter Aminobenzoesäure bzw. auf Basis von biobasiertem Anilin möglich.

Weitere Verbesserungen in der Herstellung von Anilin bzw. Anilinfolgeprodukten durch Decarboxylierung von, insbesondere fermentativ gewonnener, Aminobenzoesäure wären daher wünschenswert. Insbesondere wäre es wünschenswert, das Verfahren möglichst einfach und ohne den Einsatz von systemfremden Katalysatoren durchführen zu können, um die Wirtschaftlichkeit des Verfahrens zu erhöhen. Dies ist insbesondere erstrebenswert, da die in der Literatur als bevorzugt beschriebenen heterogenen Zeolith-Katalysatoren nicht nur einen nicht unerheblichen Kostenfaktor darstellen, sondern auch bei Einsatz im großtechnischen Produktionsmaßstab verfahrenstechnische Herausforderungen mit sich bringen. Als Beispiele für solche Herausforderungen sei insbesondere Folgendes erwähnt: Auswahl und Betrieb spezieller Reaktoren für einen heterogen katalysierten Prozess, Einbringung des Katalysators in den Prozess inklusive der Formgebung der Katalysatorpartikel, Rückhaltung des Katalysators, Massentransportlimitierungen bedingt durch den heterogenen Katalysator, Fouling des Katalysators, Deaktivierung des Katalysators über die Zeit (Katalysatorstandzeit), Entnahme des deaktivierten Katalysators (z. B. sogenannter "Ausbau" während Stillstandszeiten), Reinigung und Regeneration des heterogenen Katalysators - insbesondere außerhalb des Reaktors - und schließlich Entsorgung von nicht mehr ausreichend regenerierbarem Katalysator. Daher würde die Möglichkeit des Verzichts auf solche Katalysatoren das Verfahren insgesamt nicht nur im Hinblick auf Investitionskosten (Wegfall von Katalysatorkosten) günstiger machen, sondern auch einen zuverlässigeren, mit einem verringerten Risiko von Stillstandszeiten verbundenen, Betrieb des Verfahrens ermöglichen sowie zu einem technisch deutlich einfacheren Verfahren führen und so dessen Einsatz im großtechnischen Produktionsmaßstab attraktiver machen. Durch den Wegfall eines systemfremden heterogenen Katalysators ergeben sich bei der technischen Ausführung des Decarboxylierungsreaktors auch weitere Freiheitsgrade; zum Beispiel muss kein Konzept zur Rückhaltung des Katalysators vorgesehen werden. Für systemfremde homogene Katalysatoren können vergleichbare Argumente hervorgebracht werden. Neben den nicht unerheblichen Katalysatorkosten ist auch deren Einsatz im großtechnischen Produktionsmaßstab mit verfahrenstechnischen Herausforderungen verbunden. Beispielhaft sei insbesondere Folgendes genannt: Einbringung des Katalysators in den Reaktionsapparat, Rückhaltung bei kontinuierlicher Fahrweise und/oder Abtrennung des Katalysators aus dem Reaktionsgemisch nach der Reaktion (dies ist sowohl für kontinuierlichen als auch diskontinuierlichen Fahrweise relevant), Deaktivierung des Katalysators über die Zeit (Katalysatorstandzeit), Entnahme durch Abtrennung des deaktivierten Katalysators, Regeneration des homogenen Katalysators in einem zusätzlichen Verfahrensschritt und/oder zusätzlichen Apparat(en) und schließlich Entsorgung von nicht mehr ausreichend regenerierbarem bzw. irreversible deaktiviertem Katalysator. Die Aufarbeitung und Regeneration von homogenen Katalysatoren ist im Allgemeinen schwierig und/oder aufwändig und in vielen Fällen nicht möglich bzw. nicht wirtschaftlich.

Dem vorstehend Gesagten Rechnung tragend ist ein Gegenstand der vorliegenden Erfindung ein **Verfahren zur Herstellung von Anilin oder eines Anilinfolgeprodukts,** umfassend die folgenden Schritte:
(I) Bereitstellen einer Lösung von Aminobenzoesäure, insbesondere ortho-Aminobenzoesäure, in Anilin, wobei ein auf die Gesamtmasse aus Aminobenzoesäure und Anilin bezogener Massenanteil an Anilin in der Lösung im Bereich von 20 % bis 85 %, bevorzugt 40 % bis 80 %, besonders bevorzugt 50 % bis 75 %, eingestellt wird;
(II) Umsetzen von in der in Schritt (I) bereitgestellten Lösung enthaltenden Aminobenzoesäure zu Anilin in einem Reaktor durch thermisches Decarboxylieren bei einer Temperatur im Bereich von 165 °C bis 500 °C ohne die Anwesenheit eines systemfremden Katalysators;
(III) optional, Umsetzen von in Schritt (II) erhaltenem Anilin zu einem Anilinfolgeprodukt.

Vollkommen überraschend wurde gefunden, dass die Decarboxylierung von Aminobenzoesäure auch ohne Einsatz eines systemfremden Katalysators gelingt, wenn die Aminobenzoesäure in Anilin so gelöst wird, dass Anilin in der resultierenden Lösung in den oben genannten Anteilen zugegen ist und die Reaktion bei hinreichend hoher Temperatur durchgeführt wird. Dieses Ergebnis steht im Gegensatz zu den in der Literatur berichteten Ergebnissen, die entweder besagen, dass die Reaktion ohne systemfremden Katalysator nicht zufriedenstellend abläuft (z. B. WO 2015/124687 A1) oder die zwar von unkatalysierten Reaktionen berichten (JP 2013-230993), diese aber unter Bedingungen durchgeführt wurden, die aus anderen Gründen nachteilig sind (in der Schmelze, was verfahrenstechnisch schwierig zu beherrschen ist, oder in Wasser, was infolge der geringen Wasserlöslichkeit von Aminobenzoesäure das unter verfahrenstechnischen Gesichtspunkten unerwünschte Arbeiten mit Suspensionen sowie eine sehr energieintensive Isolierung des Produkts Anilin aus dem eingesetzten Wasser (z. B. durch Destillation) erforderlich macht, oder in alkoholischer Lösung, was den Einsatz eines systemfremden Lösungsmittels erforderlich macht).

Ohne auf eine Theorie festgelegt sein zu wollen, wird vermutet, dass Anilin in dem genannten Betriebsfenster von Konzentration und Temperatur autokatalytisch wirkt, *also seine eigene weitere Bildung aus der anwesenden Aminobenzoesäure durch Decarboxylierung katalysiert.* Daher wird die Reaktion erfindungsgemäß *"ohne die Anwesenheit eines **systemfremden** Katalysators"* durchgeführt. Unter *"systemfremden Katalysatoren"* werden der Terminologie der vorliegenden Erfindung solche Katalysatoren verstanden, die *nicht* verfahrensimmanent sind, also nicht zwangsläufig ohnehin im Verfahren zugegen sind oder durch das Verfahren gebildet werden. Die Forderung nach Abwesenheit systemfremder Katalysatoren ist daher so zu verstehen, dass der zu decarboxylierenden Lösung von Aminobenzoesäure in Anilin keine weiteren Decarboxylierungskatalysatoren (wie beispielsweise die literaturbekannten Zeolithe) zugesetzt werden. Das in der Lösung vorhandene Anilin wird dagegen als *verfahrensimmanenter* Katalysator verstanden, da Anilin das Produkt der Reaktion ist.

Erfindungsgemäß wird der *"Massenanteil an Anilin" "in der in Schritt (I) bereitgestellten Lösung"* auf "*die Gesamtmasse aus Aminobenzoesäure und Anilin"* bezogen, d. h. auf die Summe der Massen von Aminobenzoesäure und Anilin exklusive gegebenenfalls weiterer vorhandener Lösungsmittel (wie insbesondere Wasser, das in bevorzugten Ausführungsformen wie weiter unten noch näher erläutert wird vorhanden ist).

Der Begriff *"Anilinfolgeprodukt"* bezeichnet im Rahmen der vorliegenden Erfindung ein Produkt, das durch weitere chemische Umwandlung von Anilin erhalten wird.

Im Rahmen der vorliegenden Erfindung beziehen sich *pH-Werte* auf 20 °C.

### Es folgt zunächst eine Kurzzusammenfassung verschiedener möglicher Ausführungsformen der Erfindung:

In einer **ersten Ausführungsform** der Erfindung, die mit allen anderen Ausführungsformen kombiniert werden kann, werden zur Durchführung von Schritt (I) Aminobenzoesäure und Anilin in einem diskontinuierlich oder kontinuierlich betriebenen Mischer vermischt.

In einer **zweiten Ausführungsform** der Erfindung, die eine besondere Ausgestaltung der ersten Ausführungsform ist, wird als Mischer ein Rührkessel eingesetzt.

In einer **dritten Ausführungsform** der Erfindung, die eine besondere Ausgestaltung der ersten und zweiten Ausführungsform ist, werden der Mischer aus Schritt (I) und der Reaktor aus Schritt (II) kontinuierlich betrieben.

In einer **vierten Ausführungsform** der Erfindung, die eine andere besondere Ausgestaltung der ersten und zweiten Ausführungsform ist, werden zur Durchführung von Schritt (I) mehrere, insbesondere zwei, parallel geschaltete, diskontinuierlich betriebene Mischer eingesetzt, und der Reaktor aus Schritt (II) wird kontinuierlich betrieben, wobei zu jedem Zeitpunkt des kontinuierlichen Betriebs des Reaktors aus Schritt (II) aus einem der in Schritt (I) eingesetzten Mischer die Lösung der Aminobenzoesäure in den Reaktor aus Schritt (II) eingetragen wird, während in einem anderen dieser Mischer das Vermischen der Aminobenzoesäure in Anilin abläuft.

In einer **fünften Ausführungsform** der Erfindung, die mit allen anderen Ausführungsformen kombiniert werden kann, ist das thermische Decarboxylieren der Aminobenzoesäure ein erster Teilschritt (II)(1) des Schrittes (II), an welchen sich ein zweiter Teilschritt (II)(2) anschließt, in dem in Teilschritt (II)(1) gebildetes Anilin gereinigt wird, und zwar insbesondere durch Destillation in einer Destillationskolonne.

In einer **sechsten Ausführungsform** der Erfindung, die eine besondere Ausgestaltung der fünften Ausführungsform ist, wird das in Schritt (I) eingesetzte Anilin dem in Teilschritt (II)(1) gebildeten Anilin entnommen.

In einer **siebten Ausführungsform** der Erfindung, die eine andere besondere Ausgestaltung der fünften Ausführungsform ist, wird das in Schritt (I) eingesetzte Anilin dem in Teilschritt (II)(2) gereinigten Anilin entnommen.

In einer **achten Ausführungsform** der Erfindung, die eine andere besondere Ausgestaltung der fünften Ausführungsform ist, wird das in Schritt (I) eingesetzte Anilin dem in Teilschritt (II)(1) gebildeten Anilin *und* dem in Teilschritt (II)(2) gereinigten Anilin entnommen.

In einer **neunten Ausführungsform** der Erfindung, die eine besondere Ausgestaltung der sechsten und achten Ausführungsform ist, wird das in Teilschritt (II)(1) gebildete Anilin in einem Massen-Verhältnis im Bereich von 9,0: 1 bis 1 : 9,0 in zwei Ströme aufgeteilt, von denen einer der Reinigung aus Teilschritt (II)(2) zugeführt und der andere zum Bereitstellen der Lösung in Schritt (I) eingesetzt wird.

In einer **zehnten Ausführungsform** der Erfindung, die mit allen anderen Ausführungsformen kombiniert werden kann, wird in Schritt (I) Aminobenzoesäure bei einer Temperatur im Bereich von -6 °C bis 120 °C, bevorzugt 20 °C bis 120 °C, besonders bevorzugt 30 °C bis 120 °C in Anilin gelöst.

In einer **elften Ausführungsform** der Erfindung, die eine besondere Ausgestaltung der zehnten Ausführungsform ist, werden Aminobenzoesäure und Anilin zunächst bei einer Temperatur im Bereich von -6 °C bis 100 °C, bevorzugt 20 °C bis 100 °C, besonders bevorzugt 30 °C bis 120 °C, vermischt und anschließend in einer Inertgasatmosphäre auf eine Temperatur im Bereich von > 100 °C bis 120 °C erhitzt.

In einer **zwölften Ausführungsform** der Erfindung, die eine besondere Ausgestaltung der zehnten und elften Ausführungsform ist, wird die Temperatur der in Schritt (I) erhaltenen Lösung durch indirekte Beheizung in einem Wärmeaustauscher auf die Temperatur von Schritt (II) erhöht, wobei eine Verweilzeit der Lösung von Eintritt in den Wärmeaustauscher bis Eintritt in den Reaktor aus Schritt (II) im Bereich von 0,1 s bis 120 s eingehalten wird.

In einer **dreizehnten Ausführungsform** der Erfindung, die eine besondere Ausgestaltung der zehnten, elften und zwölften Ausführungsform ist, wird in Schritt (I) Aminobenzoesäure bei einem Druck im Bereich von 0,90 bar_{(abs.)} bis 3,0 bar_{(abs.)}, bevorzugt 1,0 bar_{(abs.)} bis 3,0 bar_{(abs.)}, in Anilin gelöst.

In einer **vierzehnten Ausführungsform** der Erfindung, die mit allen anderen Ausführungsformen kombiniert werden kann, wird das thermische Decarboxylieren in Schritt (II) bei einer Temperatur im Bereich von 185 °C bis 450 °C, bevorzugt im Bereich von 205 °C bis 425 °C, besonders bevorzugt im Bereich von 225 °C bis 400 °C, ganz besonders bevorzugt im Bereich von 255 °C bis 375 °C durchgeführt.

In einer **fünfzehnten Ausführungsform** der Erfindung, die mit allen anderen Ausführungsformen kombiniert werden kann, wird das thermische Decarboxylieren in Schritt (II) bei einem Druck im Bereich von 4,0 bar_{(abs.)} bis 30 bar_{(abs.)}, bevorzugt 5,0 bar_{(abs.)} bis 20 bar_{(abs.)}, besonders bevorzugt 6,0 bar_{(abs.)} bis 10 bar_{(abs.)} und ganz besonders bevorzugt 7,0 bar_{(abs.)} bis 9,0 bar_{(abs.)}, durchgeführt.

In einer **sechzehnten Ausführungsform** der Erfindung, die mit allen anderen Ausführungsformen kombiniert werden kann, wird in Schritt (II) als Reaktor ein kontinuierlich betriebener Rohrreaktor oder ein Blasensäulen-Reaktor, bevorzugt ein Blasensäulen-Reaktor, eingesetzt.

In einer **siebzehnten Ausführungsform** der Erfindung, die eine besondere Ausgestaltung der sechzehnten Ausführungsform ist, wird in Schritt (II) als Reaktor ein Blasensäulen-Reaktor eingesetzt, wobei der Blasensäulen-Reaktor Einbauten aufweist.

In einer **achtzehnten Ausführungsform** der Erfindung, die eine besondere Ausgestaltung der siebzehnten Ausführungsform ist, sind die Einbauten Lochböden, durch welche der Blasensäulen-Reaktor in Kompartimente unterteilt wird.

In einer **neunzehnten Ausführungsform** der Erfindung, die eine besondere Ausgestaltung der fünften Ausführungsform und aller davon abgeleiteten Ausführungsformen ist, werden dem Reaktor aus Schritt (II) kontinuierlich ein flüssiger, Anilin enthaltender Strom und ein gasförmiger, Kohlenstoffdioxid und gasförmiges Anilin enthaltender Strom entnommen, wobei der gasförmige Strom einen Kondensator durchläuft, in dem gasförmiges Anilin verflüssigt wird und aus dem Kohlenstoffdioxid gasförmig ausgetragen wird, wobei in dem Kondensator erhaltenes flüssiges Anilin dem Teilschritt (II)(1) und/oder dem Teilschritt (II)(2) zugeführt wird.

In einer **zwanzigsten Ausführungsform** der Erfindung, die eine besondere Ausgestaltung der neunzehnten Ausführungsform ist, wird der Druck des aus dem Kondensator gasförmig ausgetragenen Kohlenstoffdioxids auf einen Wert im Bereich von 1,0 bar_{(abs.)} bis 30 bar_{(abs.)}, bevorzugt 2,0 bar_{(abs.)} bis 20 bar_{(abs.)}, besonders bevorzugt 3,0 bar_{(abs.)} bis 10 bar_{(abs.)} und ganz besonders bevorzugt 3,0 bar_{(abs.)} bis 9,0 bar_{(abs.)}, eingestellt.

In einer **einundzwanzigsten Ausführungsform** der Erfindung, die mit allen anderen Ausführungsformen kombiniert werden kann, umfasst das Bereitstellen der Lösung von Aminobenzoesäure in Anilin in Schritt (I) die chemische Herstellung von Aminobenzoesäure.

In einer **zweiundzwanzigsten Ausführungsform** der Erfindung, die mit allen anderen Ausführungsformen kombiniert werden kann, sofern diese nicht die rein chemische Herstellung der Aminobenzoesäure zum Gegenstand haben, umfasst das Bereitstellen der Lösung von Aminobenzoesäure in Anilin in Schritt (I) folgende Teilschritte:
(I)(1) Fermentieren eines Rohstoffes, der wenigstens
   - eine fermentierbare Kohlenstoff-haltige Verbindung und
   - eine Stickstoff-haltige Verbindung

   umfasst,
   in einem Fermentationsreaktor unter Verwendung von Mikroorganismen unter Erhalt einer Aminobenzoat und/oder Aminobenzoesäure enthaltenden Fermentationsbrühe,
   Gewinnen von Aminobenzoesäure aus der Fermentationsbrühe;
   und
(I)(2) Lösen der in Schritt (I)(1) aus der Fermentationsbrühe gewonnenen Aminobenzoesäure in Anilin.

In einer **dreiundzwanzigsten Ausführungsform** der Erfindung, die eine besondere Ausgestaltung der zweiundzwanzigsten Ausführungsform ist, umfasst das Gewinnen der Aminobenzoesäure einen Schritt des Ausfällens von Aminobenzoesäure durch Säurebehandlung, wobei die dazu eingesetzte Säure Salzsäure, Schwefelsäure und/oder Phosphorsäure umfasst.

In einer **vierundzwanzigsten Ausführungsform** der Erfindung, die eine besondere Ausgestaltung der zweiundzwanzigsten Ausführungsform und aller davon abgeleiteten Ausführungsformen ist, wird das Fermentieren in Gegenwart eines Calcium-Salzes, insbesondere eines anorganischen Calcium-Salzes, durchgeführt, sodass Calcium-Aminobenzoat ausgefällt wird, wobei das Gewinnen von Aminobenzoesäure aus der Fermentationsbrühe Folgendes umfasst:
(i) Überführen von Calcium-Aminobenzoat in eine wasserlösliche Form durch Zugabe einer wässrigen Phase, die wasserlösliche Aminobenzoat-Salze bildende Kationen (insbesondere Lithium, Natrium, Kalium und/oder Ammonium-Kationen, bevorzugt Ammonium-Kationen) und wasserunlösliche Calcium-Salze bildende Anionen (insbesondere Carbonat und/oder Hydrogencarbonat-Anionen) enthält;
(ii) Abtrennen einer wässrigen Lösung von Aminobenzoat;
(iii) Einleiten von Kohlenstoffdioxid bei einem Druck von größer oder gleich 1,50 bar_{(abs.)} in die abgetrennte wässrige Lösung von Aminobenzoat, sodass eine Suspension enthaltend Aminobenzoesäure in einer wässrigen Lösung gebildet wird;
(iv) Isolieren der abgeschiedenen Aminobenzoesäure umfassend eine Druckerniedrigung unter Freisetzung von Kohlenstoffdioxid, wobei eine an Kohlenstoffdioxid abgereicherte und von abgeschiedener Aminobenzoesäure befreite wässrige Lösung erhalten wird;
(v) Verwenden der erhaltenen, an Kohlenstoffdioxid abgereicherten und von abgeschiedener Aminobenzoesäure befreiten wässrigen Lösung als Bestandteil, gegebenenfalls alleiniger Bestandteil, der in Schritt (i) zugegebenen wässrigen Phase.

In einer **fünfundzwanzigsten Ausführungsform** der Erfindung, die eine besondere Ausgestaltung der zweiundzwanzigsten Ausführungsform und aller davon abgeleiteten Ausführungsformen ist, umfasst die in Schritt (I)(1) eingesetzte fermentierbare Kohlenstoff-haltige Verbindung Stärkehydrolysat, Zuckerrohrsaft, Zuckerrübensaft, Hydrolysate aus lignocellulosehaltigen Rohstoffen oder Mischungen derselben (d. h. Mischungen aus zwei oder mehr der vorgenannten Verbindungen).

In einer **sechsundzwanzigsten Ausführungsform** der Erfindung, die eine besondere Ausgestaltung der zweiundzwanzigsten Ausführungsform und aller davon abgeleiteten Ausführungsformen ist, umfasst die in Schritt (I)(1) eingesetzte Stickstoff-haltige Verbindung Ammoniakgas, Ammoniakwasser, Ammoniumsalze (insbesondere anorganische Ammoniumsalze wie Ammoniumchlorid und/oder Ammoniumsulfat, bevorzugt Ammoniumsulfat), Harnstoff oder Mischungen derselben (d. h. Mischungen aus zwei oder mehr der vorgenannten Verbindungen).

In einer **siebenundzwanzigsten Ausführungsform** der Erfindung, die eine besondere Ausgestaltung der zweiundzwanzigsten Ausführungsform und aller davon abgeleiteten Ausführungsformen ist, umfassen die in Schritt (I)(1) eingesetzten Mikroorganismen eine Art ausgewählt aus der Gruppe bestehend aus *Escherichia coli, Pseudomonas putida, Corynebacterium glutamicum, Ashbya gossypii, Pichia pastoris, Hansenula polymorpha, Kluyveromyces marxianus, Yarrowia lipolytica, Zygosaccharomyces bailii* und *Saccharomyces cerevisiae.*

In einer **achtundzwanzigsten Ausführungsform** der Erfindung, die mit allen Ausführungsformen kombiniert werden kann, insbesondere mit der zweiundzwanzigsten Ausführungsform und allen davon abgeleiteten Ausführungsformen, umfasst die zum Bereitstellen der Lösung von Aminobenzoesäure in Anilin in Schritt (I) eingesetzte Aminobenzoesäure Wasser in einem auf die Gesamtmasse aus Aminobenzoesäure und Wasser bezogenen Massenanteil im Bereich von 0,1 % bis 40 %, bevorzugt 1,0 % bis 20 %, besonders bevorzugt 2,0 % bis 10 %.

In einer **neunundzwanzigsten Ausführungsform** der Erfindung, die mit allen Ausführungsformen kombiniert werden kann, wird Schritt (III) durchgeführt und umfasst eine der folgenden Umsetzungen:
(III)(1) säurekatalysierte Reaktion des Anilins mit Formaldehyd unter Bildung von Di- und Polyaminen der Diphenylmethanreihe;
(III)(2) säurekatalysierte Reaktion des Anilins mit Formaldehyd, gefolgt von Umsetzung mit Phosgen unter Bildung von Di- und Polyisocyanaten der Diphenylmethanreihe;
(III)(3) Umsetzung des Anilins zu einer Azoverbindung.

Die zuvor kurz geschilderten Ausführungsformen und weitere Ausgestaltungen der Erfindung werden nachfolgend näher beschrieben. Dabei können verschiedene Ausführungsformen beliebig miteinander kombiniert werden, sofern sich für den Fachmann aus dem Gesamtzusammenhang nicht das Gegenteil ergibt.

Aminobenzoesäure kommt in drei *isomeren Formen* (ortho-, meta- und para-Aminobenzoesäure) vor. Grundsätzlich kann das erfindungsgemäße Verfahren auf alle drei Isomere, entweder in isomerenreiner Form oder als Mischungen verschiedener Isomere, angewandt werden. Für alle Ausführungsformen der vorliegenden Erfindung gilt, dass die in **Schritt (I)** bereitzustellende Aminobenzoesäure vorzugsweise das ortho-Isomer umfasst. Besonders bevorzugt umfasst die in Schritt (I) zu decarboxylierende Aminobenzoesäure mindestens 50,0 mol-%, ganz besonders bevorzugt mindestens 90,0 mol-%, bezogen auf die Gesamtstoffmenge aller vorhandenen Isomere an Aminobenzoesäure, des ortho-Isomers. Außerordentlich ganz besonders bevorzugt besteht die in Schritt (I) zu decarboxylierende Aminobenzoesäure aus dem ortho-Isomer in isomerenreiner (d. h. Isomerenreinheit > 99,0 mol-%) Form.

Zur Durchführung von Schritt (I) werden Aminobenzoesäure und Anilin und Anilin vermischt. Dies geschieht vorzugsweise in einer technischen Vorrichtung zum Vermischen zweier Stoffe, einem Mischer, insbesondere einem Rührkessel. Dabei kann der Mischer diskontinuierlich oder kontinuierlich betrieben werden. Unabhängig von Art und Betriebsweise der zum Vermischen verwendeten technischen Vorrichtung wird das Vermischen so lange durchgeführt, bis die Aminobenzoesäure vollständig oder zumindest im Wesentlichen vollständig gelöst ist. Dies kann abhängig vom Massenanteil des Anilins und der gewählten Temperatur unterschiedlich lange dauern. Die Möglichkeit des Vorhandenseins eines geringfügigen Anteils nicht gelöster Aminobenzoesäure ist von der erfindungsgemäßen Terminologie *"Lösung von Aminobenzoesäure in Anilin"* umfasst. Unter einem geringfügigen Anteil wird dabei verstanden, dass höchstens 2 % der Aminobenzoesäure nicht gelöst vorliegen. Ein solch geringer Feststoffanteil ist unproblematisch, da dieser durch den fortwährenden Umsatz im Reaktor aus Schritt (II) rasch in Lösung geht und ebenfalls zu Anilin umgesetzt wird.

In einer bevorzugten Ausführungsform der Erfindung wird der Mischer kontinuierlich betrieben, d. h. ein Strom von Aminobenoesäure - als Feststoff oder Suspension in vorzugsweise wässrigem Medium - und ein Strom an Anilin werden dem Mischer kontinuierlich zugeführt, und ein Strom in Anilin gelöster Aminobenzoesäure wird dem Mischer kontinuierlich entnommen. Dies kann beispielsweise in einem dem Fachmann bekannten kontinuierlich betriebenen Rührkessel realisiert werden. Der dem Mischer kontinuierlich entnommene Aminobenzoesäure-Anilin-Strom wird kontinuierlich dem Reaktor aus Schritt (II) zugeführt.

Es ist aber auch möglich, den Mischer in Schritt (I) diskontinuierlich zu betreiben. In diesem Fall ist es bevorzugt, mehrere, insbesondere zwei, Mischer bereitzustellen, die parallel geschaltet und unabhängig voneinander regelbar sind. Dies ermöglicht es, in einem ersten Mischer Aminobenzoesäure in Anilin zu lösen, während in einem zweiten Mischer der Lösevorgang bereits abgeschlossen ist und die resultierende Lösung dem Reaktor aus Schritt (II) zugeführt werden kann. Während diese Lösung dem Reaktor kontinuierlich zugeführt wird, wird in dem ersten Mischer der Lösevorgang abgeschlossen, sodass nach Entleerung des zweiten Mischers auf den ersten Mischer umgeschaltet werden und die dort erzeugte Lösung dem Reaktor zugeführt werden kann. Die beiden Mischer sind also wechselseitig mit dem Reaktor aus Schritt (II) verbunden, sodass dieser kontinuierlich mit Aminobenzoesäure-Anilin-Lösung beschickt werden kann.

Unabhängig von der genauen verfahrenstechnischen Ausgestaltung von Schritt (I) kann dieser über einen weiten Temperaturbereich durchgeführt werden. Wesentlich ist zum einen natürlich, dass das als Lösungsmittel eingesetzte Anilin flüssig vorliegt, zum anderen aber auch, dass die Temperatur nicht so hoch ist, dass die Decarboxylierung bereits während des Lösevorgangs beginnt. Daher kann Schritt (I) bei Temperaturen im Bereich von -6 °C bis 120 °C durchgeführt werden. Bevorzugt sind Temperaturen im Bereich von 20 °C bis 120 °C, besonders bevorzugt im Bereich von 30 °C bis 120 °C. Damit ist gemeint, dass die Temperatur der vermischten Einsatzstoffe Aminobenzoesäure und Anilin in diesen Bereichen liegt, wobei die Temperatur während des Lösevorgangs innerhalb der genannten Bereiche ansteigen kann.

Es hat sich bewährt, Aminobenzoesäure und Anilin zunächst bei Temperaturen im Bereich von -6 °C bis 100 °C, bevorzugt im Bereich von 20 °C bis 100 °C, besonders bevorzugt im Bereich von 30 °C bis 100 °C zu vermischen. Dieser Schritt bedarf noch keiner Inertisierung. Die so hergestellte Mischung wird jedoch im Anschluss inertisiert, also mit einer Inertgasatmosphäre überlagert und bevorzugt auch mit einem Inertgas gestrippt, und dann in Inertgasatmosphäre auf eine Temperatur im Bereich von > 100 °C bis 120 °C erhitzt. Als Inertgas eignen sich Kohlenstoffdioxid, Edelgase wie Helium oder Argon sowie Stickstoff. Bevorzugt ist Kohlenstoffdioxid oder Stickstoff, besonders bevorzugt ist Stickstoff.

Die in Schritt (I) bereitgestellte Lösung muss noch auf die für Schritt (II) vorgesehene Temperatur erhitzt werden. Dies geschieht vorzugsweise durch indirekte Wärmeübertragung in einem Wärmeaustauscher. Hierbei ist es empfehlenswert, die Verweilzeit von Eintritt der Lösung in den Wärmeaustauscher bis zum Eintritt der aufgeheizten Lösung in den Reaktor aus Schritt (II) möglichst gering zu halten, um soweit wie möglich zu verhindern, dass die Decarboxylierungsreaktion nicht bereits im Wärmeausstauscher beginnt. Verweilzeiten im Bereich von 0,1 s bis 120 s haben sich hierzu bewährt.

Bezüglich des in Schritt (I) einzuhaltenden Drucks sind Werte im Bereich von 0,90 bar_{(abs.)} bis 3,0 bar_{(abs.)}, insbesondere 1,0 bar_{(abs.)} bis 3,0 bar_{(abs.)}, empfehlenswert, wobei der Druck im Gasraum über der Lösung gemessen wird.

Die in Schritt (I) zu bereitzustellende Aminobenzoesäure kann grundsätzlich auf jede dem Fachmann bekannte Art gewonnen werden. Eine Möglichkeit ist die Herstellung der Aminobenzoesäure **auf chemischem Wege**. Bevorzugt sind solche Verfahren, die selektiv das ortho-Isomer der Aminobenzoesäure liefern. Als eine geeignete chemische Methode sei beispielhaft die Reaktion von Phthalimid mit Natriumhypochlorit erwähnt. Phthalimid kann seinerseits aus Phthalsäureanhydrid und Ammoniak gewonnen werden. Der ganze Prozess ist wohlbekannt und wird z. B. in Lorz et al., Phthalic Acid and Derivatives in Ullmann's Encyclopedia of Industrial Chemistry, Band 27, S. 140 - 141, Weinheim, Wiley-VCH, beschrieben. Ein industrieller Prozess ist ebenfalls in der Patentliteratur beschrieben; siehe z. B. DE 29 02 978 A1 und EP 0 004 635 A2.

Die Herstellung von para-Aminobenzoesäure auf chemischem Wege kann über die Nitrierung von Toluol mit Salpetersäure, anschließende Oxidation des erhaltenen para-Nitrotoluols mit Sauerstoff zu para-Nitrobenzoesäure und schließlich Reduktion zu para-Aminobenzoesäure mit Hydrazin erfolgen. Der Gesamtprozess ist beispielsweise beschrieben in Maki et al., Benzoic Acid and Derivatives in Ullmann's Encyclopedia of Industrial Chemistry, Band 5, S. 338 ff., Weinheim, Wiley-VCH sowie in O. Kamm, et al. p-Nitrobenzoic acid in Organic Syntheses, Band 1, 1941 S. 392 ff.

Die Herstellung von meta-Aminobenzoesäure gelingt beispielsweise ausgehend von Benzoesäuremethylester: Durch Nitrierung von Benzoesäuremethylester mit Salpetersäure wird meta-Nitrobenzoesäuremethylester erhalten. Dieser Methylester wird anschließend mit Natronlauge verseift. Nach Neutralisation mit Salzsäure wird meta-Nitrobenzoesäure erhalten, die schließlich mit Hydrazin zu meta-Aminobenzoesäure reduziert wird. Das Verfahren wird beispielsweise beschrieben in Maki et al., Benzoic Acid and Derivatives in Ullmann's Encyclopedia of Industrial Chemistry Band 5, S. 338 ff., Weinheim, Wiley-VCH, in Kamm et al, Methyl m-nitrobenzoate in Organic Syntheses, Band 1, 1941, S. 372 ff. sowie in Kamm et al, m-Nitrobenzoic acid in Organic Syntheses. Band 1, 1941, S. 391 ff.

Erfindungsgemäß ist es jedoch bevorzugt, die für die Durchführung von Schritt (I) erforderliche Aminobenzoesäure durch einen **fermentativen Prozess** herzustellen. In dieser Ausführungsform der Erfindung umfasst das Bereitstellen der Lösung von Aminobenzoesäure in Anilin in Schritt (I) vorzugsweise folgende Teilschritte:
(I)(1) Fermentieren eines Rohstoffes, der wenigstens
   - eine fermentierbare Kohlenstoff-haltige Verbindung und
   - eine Stickstoff-haltige Verbindung

   umfasst,
   in einem Fermentationsreaktor unter Verwendung von Mikroorganismen unter Erhalt einer Aminobenzoat und/oder Aminobenzoesäure enthaltenden Fermentationsbrühe,
   Gewinnen von Aminobenzoesäure aus der Fermentationsbrühe;
   und
(I)(2) Lösen der in Schritt (I)(1) aus der Fermentationsbrühe gewonnenen Aminobenzoesäure in Anilin.

Schritt (I)(1) dieser Ausführungsform des erfindungsgemäßen Verfahrens kann nach einem beliebigen aus dem Stand der Technik bekannten, für die Herstellung von Aminobenzoesäure geeigneten, Fermentationsverfahren durchgeführt werden.

Je nachdem, bei welchem pH-Wert die Fermentation durchgeführt wird, fällt Aminobenzoesäure in Schritt (I)(1) nicht in der elektroneutralen Form, sondern z. B. als Aminobenzoat an (für die Art des gebildeten Isomers ist dies jedoch unerheblich). Im Rahmen dieser Erfindung wird im Zusammenhang mit Schritt (I)(1) aus Gründen der sprachlichen Vereinfachung regelmäßig von Aminobenzoesäure gesprochen, was so zu verstehen ist, dass die kationische [d. h. diprotonierte], anionische [d. h. deprotonierte] und neutrale [d. h. elektroneutrale] Form von Aminobenzoesäure mit umfasst sind. Wenn jedoch aus den Randbedingungen einer konkret geschilderten Ausführungsform hervorgeht, dass bspw. die deprotonierte Form gebildet wird, so wird von Aminobenzoat gesprochen.

Unter einer *fermentierbaren Kohlenstoff-haltigen Verbindung* im Sinne dieser Ausführungsform der vorliegenden Erfindung wird jede organische Verbindung oder Mischung organischer Verbindungen verstanden, die von den rekombinanten Zellen des eingesetzten Mikroorganismus verwendet werden kann, um Aminobenzoesäure zu produzieren. Die Produktion von Aminobenzoesäure kann dabei in Gegenwart oder Abwesenheit von Sauerstoff stattfinden. Bevorzugt sind dabei solche fermentierbaren Kohlenstoff-haltigen Verbindungen, die zusätzlich als Energie- und Kohlenstoffquelle für das Wachstum der rekombinanten Zellen des eingesetzten Mikroorganismus dienen können. Besonders geeignet sind Stärkehydrolysat, Zuckerrohrsaft, Zuckerrübensaft und Hydrolysate aus lignocellulosehaltigen Rohstoffen sowie Mischungen derselben (d. h. Mischungen aus zwei oder mehr der vorgenannten Verbindungen). Ebenfalls geeignet sind Glycerin und C1-Verbindungen, insbesondere Kohlenstoffmonoxid. Als für Schritt(I)(1) geeignete *Stickstoff-haltige Verbindungen* kommen insbesondere Ammoniakgas, Ammoniakwasser, Ammoniumsalze (insbesondere anorganische Ammoniumsalze wie Ammoniumchlorid und/oder Ammoniumsulfat, bevorzugt Ammoniumsulfat), Harnstoff oder Mischungen derselben (d. h. Mischungen aus zwei oder mehr der vorgenannten Verbindungen) in Frage.

Bevorzugte Mikroorganismen für die Durchführung von Schritt (I-0) sind **Bakterien** oder **Pilze**, und zwar insbesondere **Hefen.** Besonders bevorzugt sind dabei Mikroorganismen einer Art ausgewählt aus der Gruppe bestehend aus *Escherichia coli, Pseudomonas putida, Corynebacterium glutamicum, Ashbya gossypii, Pichia pastoris, Hansenula polymorpha, Kluyveromyces marxianus, Yarrowia lipolytica, Zygosaccharomyces bailii und Saccharomyces cerevisiae.* Ganz besonders bevorzugt bestehen die in Schritt (I)(1) eingesetzten Mikroorgansimen nur aus Vertretern genau einer dieser Arten, wobei *Corynebacterium glutamicum* ATTC 13032 außerordentlich ganz besonders bevorzugt ist. Der in der Fermentation einzuhaltende pH-Wert richtet sich nach dem eingesetzten Mikroorganismus. Mikroorganismen wie *Corynebacterium glutamicum, Pseudomonas putida* oder *Escherichia coli* werden bevorzugt bei neutralen pH-Werten (d. h. bei einem pH-Wert im Bereich von 6,0 bis 8,0) kultiviert. Mikroorganismen wie *Saccharomyces cerevisiae* werden hingegen bevorzugt im sauren Milieu kultiviert (d. h. bei einem pH-Wert im Bereich von 3,0 bis 6,0).

In jedem Fall wird der Mikroorgansimus aus Schritt (I)(1) bevorzugt so ausgewählt, dass in der Fermentation das ortho-Isomer von Aminobenzoesäure gebildet wird.

In einer bevorzugten Ausgestaltung der Erfindung werden **Bakterien als Mikroorganismen** eingesetzt. Dabei wird insbesondere auf die Patentanmeldungen WO 2015/124686 A1 und WO 2015/124687 A1 verwiesen, in denen eine erfindungsgemäß einsetzbare Fermentation unter Einsatz von Bakterien beschrieben ist (siehe beispielsweise WO 2015/124687 A1, (i) Seite 15, Zeile 8 bis Seite 16, Zeile 30, (ii) Beispiel 1 (Seite 29, Zeile 4 bis 26), (iii) Beispiel 3 (vor allem Seite 34, Zeile 10 bis 18), (iv) Beispiel 4 (vor allem Seite 55, Zeile 9 bis 31). Insbesondere kommen Bakterien zum Einsatz, die in der Lage sind, eine fermentierbare Kohlenstoff-haltige Verbindung in Gegenwart einer geeigneten Stickstoffquelle in Aminobenzoesäure umzuwandeln, ohne dass die so gebildete Aminobenzoesäure in zellinternen biochemischen Prozessen gleich wieder verbraucht wird, sodass sich Aminobenzoesäure in der Zelle anreichert und schließlich in die Fermentationsbrühe übergeht.

In einer anderen bevorzugten Ausgestaltung der Erfindung werden **Hefen als Mikroorganismen** eingesetzt. Dabei wird insbesondere auf die internationale Anmeldung WO 2017/102853 A1 verwiesen. Insbesondere kommen Hefezellen zum Einsatz, die in der Lage sind, eine fermentierbare Kohlenstoff-haltige Verbindung in Gegenwart einer geeigneten Stickstoffquelle in Aminobenzoesäure umzuwandeln, ohne dass die so gebildete Aminobenzoesäure in zellinternen biochemischen Prozessen gleich wieder verbraucht wird, sodass sich Aminobenzoesäure in der Zelle anreichert und schließlich in die Fermentationsbrühe übergeht.

Um ein derartiges Bakterium oder eine derartige Hefen zur erhalten, stehen grundsätzlich zwei Wege zur Verfügung, die in bevorzugter Ausgestaltung auch kombiniert werden können:
(i) Die enzymatischen Reaktionen im Aminobenzoesäure-Stoffwechselweg der Bakterienzelle oder Hefezelle können so erhöht werden, dass Aminobenzoesäure schneller produziert als verbraucht wird.
(ii) Die Folgereaktionen, durch welche Aminobenzoesäure in weitere Metaboliten oder Produkte (z. B. Tryptophan) überführt wird, können reduziert bzw. ausgeschaltet werden, sodass sogar die Geschwindigkeit der Aminobenzoesäure-Bildung in Wildtyp-Stämmen ausreichend ist, um zu einer Anreicherung von Aminobenzoesäure in der Zelle zu führen.

Verfahren zur Gewinnung von Bakterien oder Hefezellen mit den zuvor genannten Eigenschaften sind im Stand der Technik bekannt. Geeignete Bakterien oder Hefezellen können beispielsweise durch Screening nach Mutanten, welche Aminobenzoesäure in das umgebende Medium abgeben, identifiziert werden. Die zielgerichtete Modifikation von Schlüsselenzymen mittels gentechnischer Verfahren ist jedoch bevorzugt. Mit üblichen gentechnischen Methoden können Genexpression und Enzymaktivität nach Belieben verstärkt, verringert oder sogar ganz unterbunden werden. Es resultieren rekombinante Stämme.

Besonders bevorzugt enthalten die Bakterien oder Hefezellen, die in der Lage sind, eine fermentierbare Kohlenstoff-haltige Verbindung in Gegenwart einer Stickstoff-haltigen Verbindung zu Aminobenzoesäure umzusetzen, eine Modifizierung der Anthranilat-Phosphoribosyltransferase-Aktivität, welche besagte Enzymaktivität herabsetzt. Durch diese Modifizierung wird die Umwandlung von ortho-Aminobenzoat zu N-(5-Phospho-D-Ribosyl)-Anthranilat reduziert oder komplett unterbunden. Hierdurch wird eine Anreicherung von Aminobenzoesäure in der Zelle bewirkt. Die Bezeichnung "Anthranilat-Phosphoribosyltransferase-Aktivität" bezieht dabei sich auf eine Enzymaktivität, durch welche die Umsetzung von ortho-Aminobenzoat zu N-(5-Phospho-D-Ribosyl)-Anthranilat katalysiert wird.

In Hefen wird die Anthranilat-Phosphoribosyltransferase-Aktivität durch das native Gen TRP4 (YDR354W) genetisch kodiert. In dem Bakterium *Corynebacterium glutamicum* wird die Anthranilat-Phosphoribosyltransferase-Aktivität durch das *trpD* Gen (cg3361, Cgl3032, NCgl2929) kodiert. Im Falle von *Pseudomonas putida* erfolgt die Kodierung über das *trpD* Gen (PP_0421) innerhalb des *trpDC* Operons.

Die beschriebene Herabsetzung der Anthranilat-Phosphoribosyltransferase-Aktivität kann prinzipiell auf drei Wegen erreicht werden:
(i) Die Regulierung der Expression des Gens für die Anthranilat-Phosphoribosyltransferase-Aktivität kann so modifiziert werden, dass die Transkription des Gens oder anschließende Translation verringert oder unterbunden wird.
(ii) Die Nukleinsäuresequenz des Gens für Anthranilat-Phosphoribosyltransferase-Aktivität kann so modifiziert werden, dass das Enzym, welches durch das modifizierte Gen kodiert wird, eine geringere spezifische Aktivität aufweist.
(iii) Das native Gen für Anthranilat-Phosphoribosyltransferase-Aktivität kann durch ein anderes Gen, das von einem verschiedenen Organismus stammt, ersetzt und ein Enzym mit einer spezifischen Anthranilat-Phosphoribosyltransferase-Aktivität, die geringer ist als die der zuvor erwähnten nativen Gene (z.B. TRP4, *trpD* oder *trpDC*)*,* kodiert werden.

Unabhängig davon, welcher Mikroorganismus eingesetzt wird, umfasst die Fermentationsbrühe zu Beginn der Fermentation in Schritt (I)(1) rekombinante Zellen des eingesetzten Mikroorganismus und mindestens eine fermentierbare Kohlenstoff-haltige Verbindung (sowie mindestens eine Stickstoff-haltige Verbindung als Stickstoffquelle). Bevorzugt enthält die Fermentationsbrühe darüber hinaus noch weitere Bestandteile ausgewählt aus der Gruppe bestehend aus Puffersystemen, anorganischen Nährstoffen, Aminosäuren, Vitaminen und weiteren organischen Verbindungen welche für das Wachstum bzw. den Erhaltungsstoffwechsel der rekombinanten Zellen benötigt werden. Die Fermentationsbrühe ist wasserbasiert. Nach Einsetzen des Fermentationsprozesses umfasst die Fermentationsbrühe auch Aminobenzoesäure, das angestrebte Fermentationsprodukt.

Bevorzugt umfasst der Schritt (I)(1) auch eine *Aufarbeitung der erhaltenen Fermentationsbrühe.* Diese Aufarbeitung umfasst bevorzugt die folgenden Schritte:
(α) Abtrennung des Mikroorganismus aus der Fermentationsbrühe
und/oder
(β) Entfärbung der Fermentationsbrühe oder, bei Durchführung von Schritt (a), der in Schritt (α) erhaltenen an Mikroorganismen abgereicherten Fermentationsbrühe.

Die *Abtrennung des Mikroorganismus aus der Fermentationsbrühe* in Schritt (α) ist an sich aus dem Stand der Technik bekannt und erfolgt im Rahmen der vorliegenden Erfindung insbesondere durch Filtration, Absetzen (sog. Settling), Abtrennung in Hydrozyklonen oder Zentrifugation. Eine mögliche Ausgestaltung dieses Schrittes ist in WO 2015/124686 A1 und WO 2015/124687 A1 beschrieben. Dabei wird insbesondere auf WO 2015/124687 A1, Seite 15, Zeile 8 bis Seite 15, Zeile 17, verwiesen.

Unabhängig davon, ob der Mikroorganismus abgetrennt wird oder nicht, kann Schritt (I)(1) erforderlichenfalls einen Schritt (β) zur *Entfärbung der Fermentationsbrühe bzw. der an Mikroorganismen abgereicherten Fermentationsbrühe* umfassen. Dieser Schritt (β) wird bevorzugt so durchgeführt, dass Fermentationsbrühe bzw. an Mikroorganismen abgereicherte Fermentationsbrühe über eine Säule mit fester Packung geleitet wird, um Farbstoffe mittels Adsorption zu entfernen. Als mögliche feste Phase kann z. B. Kieselgur oder Ionenaustauscherpackungen verwendet werden. Schritt (β) wird bevorzugt dann durchgeführt, wenn in der Fermentationsbrühe bzw. der an Mikroorganismen abgereicherten Fermentationsbrühe aus Schritt (α) solche farbigen Substanzen vorhanden sind, welche den Schritt der *Gewinnung der Aminobenzoesäure aus der Fermentationsbrühe* stören könnten.

Der Schritt der *Gewinnung der Aminobenzoesäure* aus der, gegebenenfalls gemäß Schritt (α) und/oder Schritt (β) behandelten, Fermentationsbrühe, kann abhängig vom pH-Wert der erhaltenen Fermentationsbrühe unterschiedlich ausgestaltet werden.

In der Mehrzahl der Fälle ist die Fermentationsbrühe nach Schritt (I)(1) basisch bis neutral oder allenfalls leicht sauer (pH > 4,7), und die Aminobenzoesäure liegt infolgedessen in Form ihres Anions Aminobenzoat gelöst vor. In diesen Fällen ist es bevorzugt, die Fermentationsbrühe mit Säure zu behandeln, und zwar insbesondere mit Salzsäure, Schwefelsäure und/oder Phosphorsäure, um das Anion in die elektroneutrale Form zu überführen. Die Säurezugabe erfolgt dabei insbesondere solange, bis der pH-Wert der resultierenden Mischung im Bereich von 3,0 bis 4,7, bevorzugt im Bereich von 3,2 bis 3,7 (insbesondere bei meta- und para-Aminobenzoesäure), besonders bevorzugt im Bereich von 3,4 bis 3,6 (insbesondere bei ortho-Aminobenzoesäure), liegt. Dann liegt Aminobenzoesäure überwiegend bis vollständig in der elektroneutralen Form vor und fällt infolge der geringen Wasserlöslichkeit derselben bis auf einen kleinen, auf eine gewisse Restlöslichkeit zurückzuführenden Anteil, aus und kann leicht von der überstehenden Fermentationsbrühe abgetrennt werden, insbesondere durch Filtration oder Zentrifugation. Eine Filtration kann bei vermindertem Druck, Umgebungsdruck oder erhöhtem Druck durchgeführt werden. Eine Zentrifugation kann mit handelsüblichen Zentrifugen durchgeführt werden. Es ist auch möglich, die in der Säurebehandlung erhaltene Suspension ruhen zu lassen, bis sich die ausgefallenen Kristalle an Aminobenzoesäure absetzen, und dann die überstehende Mutterlauge abzudekantieren oder abzusaugen.

Sollte die Fermentationsbrühe nach Schritt (I)(1) stark sauer (pH < 3,0) sein, wird ein pH-Wert in den vorgenannten Bereichen durch Zugabe von Base (bevorzugt Natronlauge, Kalk) sichergestellt. Liegt der pH-Wert der Fermentationsbrühe nach Schritt (I)(1), gegebenenfalls nach Durchführung von Schritt (α) und/oder Schritt (β), dagegen im Bereich von 3,0 bis 4,0, wie es beim Einsatz von Hefen als Mikroorganismen der Fall sein kann, wird in einer bevorzugten Ausführungsform weder Säure noch Base zugegeben, sondern die Fermentationsbrühe wird direkt ohne weitere pH-Wert-Anpassung weiter verarbeitet. In diesem Fall ist damit zu rechnen, dass Kristalle von Aminobenzoesäure spontan ausfallen und direkt abgetrennt werden können. Bezüglich der hierzu anwendbaren Methoden gilt das oben bei der Säurebehandlung Gesagte entsprechend.

Eine erforderlichenfalls nötige Trennung von in wässriger Lösung vorliegender fester Aminobenzoesäure und festen Mikroorganismen gelingt am besten durch Zentrifugation. Dies gilt für alle Ausführungsformen der vorliegenden Erfindung, in denen eine solche Trennung erforderlich ist.

Die auf eine der vorstehend beschriebenen Weisen erhaltene Aminobenzoesäure kann vor der Durchführung von Schritt (I)(2) weiter aufbereitet werden. Bevorzugt ist eine Wäsche mit wässrigen Waschmedien, insbesondere Wasser. Um Ausbeuteverluste zu vermeiden, kann der pH-Wert des wässrigen Waschmediums auf den gleichen Wert wie nach beendeter Säurezugabe (bzw. im Fall von Hefen: Basenzugabe) eingestellt werden; es wird also in dieser Ausführungsform statt mit Wasser mit einer verdünnten Säure gewaschen. Als Säuren hierfür eignen sich die oben im Zusammenhang mit der Säurebehandlung genannten Säuren.

In einer bevorzugten Ausführungsform wird Schritt (I)(1) kontinuierlich durchgeführt, d. h. dass die Edukte dem Fermentationsreaktor kontinuierlich zugeführt und das Produkt dem Fermentationsreaktor kontinuierlich entnommen wird. In kontinuierlicher Verfahrensführung wird der Mikroorganismus unter Umständen mit dem Produktstrom ausgetragen; der Mikroorganismus reproduziert sich jedoch im Allgemeinen selbst, sodass eine Zuführung von frischem Mikroorganismus in der Regel nicht nötig ist (erforderlichenfalls aber natürlich vorgenommen werden kann). Eine Zellrückhaltung zur Vermeidung der Austragung von Mikroorganismus ist ebenfalls möglich.

In einer anderen bevorzugten Ausführungsform wird Schritt (I)(1) in einer diskontinuierlichen Verfahrensführung (sog. "Batch-Fahrweise") durchgeführt. In einer Variante der diskontinuierlichen Fahrweise (sog. "*Fed*-Batch-Fahrweise") werden die Edukte dem Fermentationsreaktor so lange kontinuierlich zugeführt, wie es das Reaktorvolumen erlaubt, ohne dass Produkte dem Reaktor entnommen werden. Die Reaktion wird nach Zugabe der maximal möglichen Menge an Edukten unterbrochen und das Produktgemisch dem Fermentationsreaktor entnommen.

Unabhängig von der genauen Fahrweise umfasst der Fermentationsreaktor bevorzugt Einrichtungen zur Messungen wichtiger Prozessparameter wie Temperatur, pH-Wert der Fermentationsbrühe, Konzentration an Substrat und Produkt, Gehalt an gelöstem Sauerstoff, Zelldichte der Fermentationsbrühe. Insbesondere bevorzugt umfasst der Fermentationsreaktor Einrichtungen zur Anpassung von mindestens einem (bevorzugt von allen) der vorgenannten Prozessparameter.

Geeignete Fermentationsreaktoren sind Rührkessel, Membranreaktoren, Kolbenstromreaktoren ("plug flow reactors") oder Schlaufenreaktoren (siehe beispielsweise Bioprozesstechnik, Horst Chmiel, ISBN-10: 3827424763, Spektrum Akademischer Verlag). Besonders bevorzugt sowohl für aerobe als auch für anaerobe Fermentationen sind Rührkesselreaktoren und Schlaufenreaktoren (insbesondere Airliftreaktoren, in denen die Zirkulation der Flüssigkeit im Reaktor durch Begasung erreicht wird).

In einer besonders bevorzugten Ausführungsform wird Schritt (I)(1) in Gegenwart eines Calcium-Salzes durchgeführt, sodass Aminobenzoesäure in Form ihres schwerlöslichen Calcium-Salzes Ca(OOC-C₆H₄-NH₂)₂ ausfällt. Ein solches Verfahren ist in der noch unveröffentlichten europäischen Patentanmeldung EP18176433.3 beschrieben und umfasst die folgenden weiteren Schritte:
(i) Überführen von Calcium-Aminobenzoat in eine wasserlösliche Form durch Zugabe einer wässrigen Phase, die wasserlösliche Aminobenzoat-Salze bildende Kationen (insbesondere Lithium, Natrium, Kalium und/oder Ammonium-Kationen, bevorzugt Ammonium-Kationen) und wasserunlösliche Calcium-Salze bildende Anionen (insbesondere Carbonat und/oder Hydrogencarbonat-Anionen) enthält;
(ii) Abtrennen einer wässrigen Lösung von Aminobenzoat;
(iii) Einleiten von Kohlenstoffdioxid bei einem Druck von größer oder gleich 1,50 bar_{(abs.)} in die abgetrennte wässrige Lösung von Aminobenzoat, sodass eine Suspension enthaltend Aminobenzoesäure in einer wässrigen Lösung gebildet wird;
(iv) Isolieren der abgeschiedenen Aminobenzoesäure umfassend eine Druckerniedrigung unter Freisetzung von Kohlenstoffdioxid, wobei eine an Kohlenstoffdioxid abgereicherte und von abgeschiedener Aminobenzoesäure befreite wässrige Lösung erhalten wird;
(v) Verwenden der erhaltenen, an Kohlenstoffdioxid abgereicherten und von abgeschiedener Aminobenzoesäure befreiten wässrigen Lösung als Bestandteil, gegebenenfalls alleiniger Bestandteil, der in Schritt (i) zugegebenen wässrigen Phase.

Unabhängig vom eingesetzten Mikroorganismus und der gewählten Kohlenstoff- und Stickstoffquelle wird das in Schritt (I)(1) einzusetzende Calcium-Salz bevorzugt ausgewählt aus Calciumcarbonat, Calciumhydrogencarbonat, Calciumhydroxid, Calciumoxid oder Mischungen aus zwei oder mehr der vorgenannten Verbindungen. Insbesondere bevorzugt ist der Einsatz einer Mischung aus Calciumcarbonat und Calciumhydroxid. Eine Suspension von Calciumcarbonat in Wasser enthält - da Calciumcarbonat zu einem Teil in Lösung geht und die gelösten Carbonationen mit Wasser Hydrogencarbonat- und Hydroxidionen ausbilden - immer auch Anteile an Calciumhydroxid und ist daher von der Formulierung "Mischung aus Calciumcarbonat und Calciumhydroxid" umfasst. Der Einsatz solcher Calcium-Salze hat den Vorteil, dass die Zugabe von weiteren Basen wie beispielsweise Natriumhydroxid (siehe etwa die in WO 2015/124686 A1 und WO 2015/124687 A1 beschriebenen Verfahren) als Puffer nicht mehr oder allenfalls in verringerter Menge erforderlich ist. Calciumcarbonat kann dabei in fester Form im Fermentationsreaktor vorgelegt werden. Eine Zugabe als wässrige Suspension ist auch möglich. Calciumoxid kann grundsätzlich ebenfalls in fester Form in den Fermentationsreaktor eingeführt werden. Steht Calciumoxid als Calciumquelle zur Verfügung, so ist es jedoch bevorzugt, dieses zunächst mit Wasser abzulöschen und so in Calciumhydroxid zu überführen. Calciumhydroxid und Calciumhydrogencarbonat werden vorzugsweise in Form von wässrigen Lösungen zudosiert.

Die nach der Isolierung des Calcium-Aminobenzoats verbleibende wässrige Fermentationslösung weist einen pH-Wert von insbesondere 4,0 oder mehr auf, abhängig von den genauen Bedingungen der Fermentation. Aus dieser wässrigen Fermentationslösung kann, da sie noch Anteile gelösten Aminobenzoats enthalten kann, in vorteilhafter Weise durch Säurezugabe bis Aminobenzoesäure auskristallisiert und die auskristallisierte Aminobenzoesäure isoliert werden, wobei eine an Aminobenzoesäure abgereicherte Mutterlauge verbleibt. Bezüglich geeigneter Säuren und pH-Werte sei auf die obigen Ausführungen verwiesen, die auch hier gelten.

Unabhängig von der genauen Ausgestaltung der Fermentation und des Schrittes des Gewinnens der Aminobenzoesäure fällt dieselbe bei fermentativer Gewinnung stets wasserhaltig an. Es ist bevorzugt, diesen Wassergehalt nicht zu entfernen oder zumindest nicht vollständig zu entfernen, sodass die zum Bereitstellen der Lösung von Aminobenzoesäure in Anilin in Schritt (I) eingesetzte Aminobenzoesäure Wasser in einem auf die Gesamtmasse aus Aminobenzoesäure und Wasser bezogenen Massenanteil im Bereich von 0,1 % bis 40 %, bevorzugt 1,0 % bis 20 %, besonders bevorzugt 2,0 % bis 10 %, umfasst. Dies hat den Vorteil, dass auf die vollständige Trocknung von wasserfeuchter Aminobenzoesäure, die sehr energie- und zeitaufwendig ist und spezieller technischer Apparate bedarf, wegfällt. Auf diese vollständige Trocknung kann verzichtet werden, was den Zeitaufwand und die Kosten sowie die technische Komplexität des Gesamtverfahrens deutlich reduziert.

Die auf die vorstehend beschriebene Weise bereitgestellte Lösung von Aminobenzoesäure in Anilin wird in **Schritt (II)** Decarboxylierungsbedingungen unterworfen. Erfindungsgemäß sind hierzu hohe Temperaturen erforderlich, d. h. Temperaturen im Bereich von 165 °C bis 500 °C. Bevorzugt sind Temperaturen im Bereich von 185 °C bis 450 °C, besonders bevorzugt im Bereich von 205 °C bis 425 °C, ganz besonders bevorzugt im Bereich von 225 °C bis 400 °C, außerordentlich ganz besonders bevorzugt im Bereich von 255 °C bis 375 °C. Hinsichtlich des Verfahrensdrucks ist es bevorzugt, das thermische Decarboxylieren in Schritt (II) bei Druckwerten im Bereich von 4,0 bar_{(abs.)} bis 30 bar_{(abs.)}, bevorzugt 5,0 bar_{(abs.)} bis 20 bar_{(abs.)}, besonders bevorzugt 6,0 bar_{(abs.)} bis 10 bar_{(abs.)} und ganz besonders bevorzugt 7,0 bar_{(abs.)} bis 9,0 bar_{(abs.)}, durchzuführen. Hiermit ist der Druck im Gasraum des Reaktors oberhalb des Flüssigkeitsspiegels gemeint (im Falle von aufrecht angeordneten kolonnenförmigen Reaktoren der Druck im Kopf des Reaktors).

Für die meisten Anwendungen ist es vorzuziehen, dass in der Decarboxylierung gebildete Anilin vor dessen weiterer Verwendung zu reinigen, insbesondere durch Destillation. In diesem Fall umfasst der Schritt (II) der vorliegenden Erfindung einen ersten Teilschritt (II)(1), die thermische Decarboxylierung, an welchen sich ein zweiter Teilschritt (II)(2) anschließt, in dem in Teilschritt (II)(1) gebildetes Anilin gereinigt wird. Diese Reinigung kann durch dem Fachmann geläufige Verfahren erfolgen. Insbesondere umfasst die Reinigung mindestens einen Destillationsschritt, welchem eine Wasserabtrennung durch Phasentrennung vorgeschaltet sein kann. Die Reinigung kann ebenfalls eine Basenbehandlung zur Entfernung saurer Verunreinigungen vor, während oder nach dem Destillationsschritt umfassen. Geeignete Ausgestaltungen sind beispielsweise in EP-A-1 845 079, EP-A-1 845 080, EP-A-2 263 997 und EP-A-2 028 176 beschrieben. (Diese Schriften befassen sich mit der Reinigung von Anilin, das durch Hydrierung von Nitrobenzol erhalten wurde; die beschriebenen Reinigungsschritte des Rohanilins sind jedoch auf anders hergestelltes Anilin ebenfalls anwendbar.)

Das in Schritt (I) für das Auflösen der Aminobenzoesäure einzusetzende Anilin kann dem in Teilschritt (II)(1) gebildeten Anilin entnommen werden, d. h., die Aminobenzoesäure wird in Schritt (I) in *Roh*anilin gelöst. Die Verwendung von Rohanilin als Lösungsmittel für zu decarboxylierende Aminobenzoesäure ist bereits aus der internationalen Patentanmeldung WO 2018/002088 A1 bekannt. Sofern auf den dort geforderten systemfremden Katalysator verzichtet wird und die erfindungsgemäß geforderten Wertebereiche für Temperatur und Anilinmassenanteil eingehalten werden, kann auf die dort beschriebene verfahrenstechnische Vorgehensweise in vorteilhafter Weise zurückgegriffen werden.

Es ist jedoch ebenfalls möglich, das in Schritt (I) für das Auflösen der Aminobenzoesäure einzusetzende Anilin dem in Teilschritt (II)(2) gebildeten Anilin zu entnehmen, also *Rein*anilin für das Auflösen der Aminobenzoesäure in Schritt (I) einzusetzen. Dies ist insbesondere dann bevorzugt, wenn das Rohanilin die Decarboxylierung beeinträchtigende Verunreinigungen in einem problematischen Umfang enthalten sollte. Daneben kann selbstverständlich auch eine Mischung aus Roh- und Reinanilin, also Anilin aus den Teilschritten (II)(1) und (II)(2), zum Auflösen der Aminobenzoesäure in Schritt (I) zum Einsatz kommen, etwa um solche Verunreinigungen in einen unkritischen Bereich hinein zu verdünnen.

Sofern Rohanilin für die Durchführung von Schritt (I) zum Einsatz kommen soll, ist es bevorzugt, das in Teilschritt (II)(1) gebildete Anilin in einem Massen-Verhältnis im Bereich von 9,0 : 1 bis 1 : 9,0 in zwei Ströme aufzuteilen, von denen einer der Reinigung aus Teilschritt (II)(2) zugeführt und der andere zum Bereitstellen der Lösung in Schritt (I) eingesetzt wird.

Während des thermischen Decarboxylierens entsteht gasförmiges Kohlenstoffdioxid. Es ist bevorzugt, dieses dem in Schritt (II) eingesetzten Reaktor kontinuierlich zu entnehmen. Dabei wird immer auch ein Teil des Anilins gasförmig mitgerissen werden, sodass es sich zur Vermeidung von Produktverlusten empfiehlt, den ausgeschleusten, Kohlenstoffdioxid und gasförmiges Anilin enthaltenden Gasstrom durch einen Kondensator zu führen, in dem gasförmiges Anilin verflüssigt und aus dem Kohlenstoffdioxid gasförmig ausgetragen wird, wobei in dem Kondensator erhaltenes flüssiges Anilin dem Teilschritt (II)(1) und/oder dem Teilschritt (II)(2) zugeführt wird. Es ist bevorzugt, den Druck des aus dem Kondensator gasförmig ausgetragenen Kohlenstoffdioxids auf einen Wert im Bereich von 1,0 bar_{(abs.)} bis 30 bar_{(abs.)}, bevorzugt 2,0 bar_{(abs.)} bis 20 bar_{(abs.)}, besonders bevorzugt 3,0 bar_{(abs.)} bis 10 bar_{(abs.)} und ganz besonders bevorzugt 3,0 bar_{(abs.)} bis 9,0 bar_{(abs.)}, einzustellen. Dies kann auf einfache Weise mittels dem Fachmann bekannter Regelstrecken erreicht werden.

Als *Reaktoren* für die Durchführung von Schritt (II) eignen sich grundsätzlich in der Verfahrenstechnik übliche, dem Fachmann geläufige Reaktortypen. Der Ausdruck *"in einem Reaktor"* umfasst dabei erfindungsgemäß auch Ausführungsformen, in denen mehrere Reaktoren zu einer Reaktorkaskade hintereinandergeschaltet sind, d. h. der flüssige Produktaustrag eines Reaktors fließt zur weiteren Umsatzvervollständigung in den nächstfolgenden Reaktor. Es ist möglich, nur in den ersten Reaktor einer Reaktorkaskade die in Schritt (I) bereitgestellte Lösung von Aminobenzoesäure in Anilin einzuspeisen. Es ist jedoch ebenfalls möglich, in jeden Reaktor einer Reaktorkaskade die in Schritt (I) bereitgestellte Lösung einzuspeisen.

In einer Ausführungsform der Erfindung wird in Schritt (II) als Reaktor ein kontinuierlich betriebener Rohrreaktor eingesetzt.

In einer anderen Ausführungsform der Erfindung wird in Schritt (II) als Reaktor ein kontinuierlich betriebener Blasensäulen-Reaktor eingesetzt. Dieser weist bevorzugt Einbauten auf, und zwar insbesondere Lochböden, durch welche der Blasensäulen-Reaktor in Kompartimente unterteilt wird. *Blasensäulen* sind Apparate, in denen Gas in Form von Blasen mit einer kontinuierlich strömenden Flüssigkeit in Kontakt tritt. Im Rahmen der vorliegenden Erfindung ist die kontinuierlich strömende Flüssigkeit die Lösung von Aminobenzoesäure in Anilin, die einen aufrecht angeordneten kolonnenförmigen Reaktor vorzugsweise von unten nach oben durchströmt. Das Gas ist das gebildete Kohlenstoffdioxid (sowie gegebenenfalls zusätzlich zugeführtes Inertgas). Im Kopf des Reaktors findet eine Flüssig-Gas-Trennung statt; flüssiges Anilin trennt sich von Kohlenstoffdioxid-Gas (das gegebenenfalls Anteile mitgerissenen gasförmigen Anilins enthält).

In allen Ausführungsformen ist es bevorzugt, Schritt (II) unter Sauerstoffausschluss durchzuführen. Zur Inertisierung des Reaktors eignen sich inerte Gase wie Stickstoff, Kohlenstoffdioxid oder Edelgase wie Helium oder Argon. Bevorzugt ist Stickstoff oder Kohlenstoffdioxid, besonders bevorzugt ist Stickstoff.

Die erfindungsgemäße Vorgehensweise, die Decarboxylierung in einem Reaktionsmedium durchzuführen, das in wesentlichen Anteilen aus Anilin besteht und keinen systemfremden Katalysator umfasst, hat gegenüber den literaturbekannten Verfahren vielfältige Vorteile:
- Der Verzicht auf systemfremde Katalysatoren reduziert die Komplexität des Verfahrens erheblich, wie weiter oben detailliert erläutert wurde;
- Reduzierte Kosten, da keine Kosten für Beschaffung und Regeneration von Katalysatoren zu Buche schlagen;
- Vereinfachte Aufreinigung des Produkts Anilin, da kein Katalysator abgetrennt werden muss.

Das erfindungsgemäß gewonnene Anilin wird bevorzugt zu einem *Anilinfolgeprodukt* weiter umgesetzt, d. h. **Schritt (III)** wird bevorzugt durchgeführt. Ausgewählte weitere Umsetzungen des erhaltenen Anilins in Schritt (III) sind:
(III)(1) säurekatalysierte Reaktion des Anilins mit Formaldehyd unter Bildung von Di- und Polyaminen der Diphenylmethanreihe;
(III)(2) säurekatalysierte Reaktion des Anilins mit Formaldehyd, gefolgt von Umsetzung mit Phosgen unter Bildung von Di- und Polyisocyanaten der Diphenylmethanreihe;
(III)(3) Umsetzung des Anilins zu einer Azoverbindung.

Die weitere Umsetzung Anilins mit Formaldehyd zu **Di- und Polyaminen der Diphenylmethanreihe (III)(1)** ist an sich bekannt und kann nach einem beliebigen Verfahren des Standes der Technik durchgeführt werden. Die kontinuierliche oder teilweise diskontinuierliche Herstellung von Di- und Polyaminen der Diphenylmethanreihe aus Anilin und Formaldehyd ist z. B. in EP 1 616 890 A1, US-A-5286760, EP-A-451442 und WO-A-99/40059 offenbart. Die Reaktion erfolgt unter Säurekatalyse. Als saurer Katalysator eignet sich vorzugsweise Salzsäure.

Die weitere Umsetzung der so erhaltenen Di- und Polyamine der Diphenylmethanreihe mit Phosgen zu **Di- und Polyisocyanaten der Diphenylmethanreihe (III)(2)** ist ebenfalls an sich bekannt und kann nach einem beliebigen Verfahren des Standes der Technik durchgeführt werden. Geeignete Verfahren sind beispielsweise in EP 2 077 150 B1, EP 1 616 857 A1, EP 1 873 142 A1, und EP 0 314 985 B1 beschrieben.

Die Umsetzung des erfindungsgemäß erhaltenen Anilins zu **Azoverbindungen, insbesondere zu Azo-Farbstoffen (III)(3)** kann nach einem beliebigen Verfahren des Standes der Technik erfolgen. Beispielhaft sei auf die bekannte Herstellung von Anilingelb (para-Aminoazobenzol; CAS 493-5-7) oder Indigo (2,2'-Bis(2,3-dihydro-3-oxomethyliden); CAS 482-89-3) verwiesen (Per Wiklund et al., Current Organic Synthesis, 2006, 3, 379 - 402).

### Beispiele

### Eingesetzte Chemikalien:

2-Aminobenzoesäure, CAS 118-92-3 (nachfolgend kurz: oAB): Reinheit ≥ 98 %, Sigma-Aldrich Chemie GmbH.

Anilin, CAS 62-53-3 (nachfolgend kurz: ANL): Reinheit ≥ 99 %, Sigma-Aldrich Chemie GmbH.

VE-Wasser (nachfolgend kurz: H₂O): Reinheit "HPLC Grade", Sigma-Aldrich Chemie GmbH.

2-Amino-N-phenylbenzamid, CAS 4424-17-3 (nachfolgend kurz: Amid): Reinheit ≥ 98 %, Sigma-Aldrich Chemie GmbH.

Methanol, CAS 67-56-1 (nachfolgend kurz: MeOH): Reinheit "HPLC Grade", Sigma-Aldrich Chemie GmbH.

Phosphorsäure "ACS reagent", CAS 7664-38-2 (nachfolgend kurz: H₃PO₄): Reinheit ≥ 85 %, Sigma-Aldrich Chemie GmbH.

### Katalysator (für Vergleichsbeispiele):

CBV 600 (CAS 1318-02-1), Zeolyst International, Inc., Oberfläche 660 m²/g, Porengröße 2,43 nm, Si/Al-Verhältnis 2,5. Der Katalysator wurde vor Verwendung 3 h bei 300 °C in Luft kalziniert.

### Allgemeine Versuchsvorschrift für Versuche mit Katalysator (Vergleichsbeispiele):

1,33 g oAB, 2 mL ANL und 0,08 g Katalysator wurden in einem 10 mL-Druckreaktor vorgelegt, mit Argon als Schutzgas gespült, und der Reaktor wurde verschlossen. Anschließend wurde ein Argon-Druck von 3 bar beaufschlagt, 2 min bei 800 U/min gerührt und der Druck auf 1 bar abgelassen. Dieser Vorgang wurde noch 3-mal wiederholt, bevor der Reaktor auf die Reaktionstemperatur (siehe Tabelle 1) gebracht wurde. Nach der entsprechenden Reaktionszeit (siehe Tabelle 1) wurde der Druckreaktor von Reaktionstemperatur auf Raumtemperatur im Eisbad abgekühlt und der Druck anschließend abgelassen. Der Katalysator wurde mittels Zentrifugation aus der Reaktionsmischung abgetrennt (5 min, 5000 U/min). Die Zusammensetzung des flüssigen Überstandes wurde mittels HPLC-Analytik untersucht (Daten siehe Tabelle 1).

### Allgemeine Versuchsvorschrift für Versuche ohne Katalysator (Ausführungsbeispiele):

Die Reaktionsdurchführung erfolgte analog zu "Allgemeine Versuchsvorschrift für Versuche mit Katalysator", mit der Ausnahme, dass ohne Katalysator gearbeitet wurde und Versuchsschritte, die den Katalysator betreffen somit entfallen sind.

### HPLC-Messunsen:

Zur quantitativen Bestimmung von oAB, ANL und Amid im Reaktionsgemisch wurde Hochdruckflüssigkeitschromatographie (HPLC)-Analytik durchgeführt. Für die HPLC-Analytik wurde ein Setup der Firma Agilent verwendet mit UV-Detektion (DAD, gemessen bei 254,4 nm). Zur Trennung wurde eine Säule von Agilent (Eclipse XDB-C18; 5 µm; 4,6 x 150 mm) verwendet. Als Fließmittel wurde eine Mischung aus MeOH und H₂O verwendet (Verhältnis 40:60, pH = 3 mit H₃PO₄ eingestellt) bei einer Flussrate von 0,7 mL/min. Die Temperatur des Säulenofens betrug 25 °C. Die Probe wurde im Verhältnis 1 : 10 in MeOH verdünnt, das Injektionsvolumen betrug 1 µL. Die Retentionszeiten der Einzelkomponenten ANL, oAB und Amid betrugen:
- ANL = 2,87 min;
- oAB = 6,00 min;
- Amid = 18,67 min.

Die Peakflächen werden in Flächenprozent (A%) umgerechnet. Die Quantifizierung der Einzelkomponenten in Massenprozent (Ma.-%), bezogen auf das Reaktionsgemisch, wurde durch eine zuvor erstellte Kalibrierung mit Reinsubstanzen ermöglicht. In Tabelle 1 sind alle Produktzusammensetzungen für die Ausführungs- und Vergleichsbeispiele bei gegebener Reaktionszeit tabelliert.

### Bestimmung der Reaktionsgeschwindigkeiten:

Zur Bestimmung der Reaktionsgeschwindigkeit wurde die Geschwindigkeitskonstante k (in min⁻¹) des Umsatzes an oAB ermittelt. Dazu wurde eine Umsatzkinetik pseudo-erster Ordnung bzgl. oAB zugrunde gelegt und der Umsatz von oAB für verschiedene Reaktionszeiten experimentell bestimmt. Zur Berechnung von k wurden die natürlichen Logarithmen der relativen oAB-Konzentrationen gegen die Reaktionszeit (in min) aufgetragen und linear angepasst. Die Steigung der so gewonnenen Geradengleichung entspricht k in min⁻¹. In Tabelle 1 sind alle k-Werte für die Ausführungs- und Vergleichsbeispiele tabelliert.

**Tabelle 1: Ausführungsbeispiele (ohne Katalysator) und Vergleichsbeispiele (mit Katalysator)**

| # | Anmerkung | T[°C] | k [min⁻¹] | Zusammensetzung Produktgemisch | | | | |
|---|---|---|---|---|---|---|---|---|
| | | | | nach [min] | oAB [Ma.-%] | ANL [Ma.-%] | Amid [Ma.-%] | ΣA% dieser Komponenten in der HPLC |
| 1 | ohne Katalysator | 200 | 0,053 | 60 | 10,9 | 88,9 | 0,2 | 100 |
| 2 | | 230 | 0,091 | 60 | 3,2 | 96,5 | 0,3 | 100 |
| 3 | | 250 | 0,118 | 30 | 0,8 | 98,8 | 0,4 | 100 |
| 4 | | 270 | 0,143 | 30 | 0,4 | 99,2 | 0,4 | 100 |
| 5 | ohne Katalysator | 200 | 0,032 | 60 | 14,1 | 85,7 | 0,3 | 100 |
| 6 | mit 5 Ma.-% H₂O bezogen auf oAB | 225 | 0,052 | 60 | 3,9 | 95,7 | 0,3 | 100 |
| 7 | ohne Katalysator | 200 | 0,031 | 60 | 14,1 | 85,7 | 0,2 | 100 |
| 8 | mit 30 Ma.-% H₂O bezogen auf oAB | 225 | 0,054 | 60 | 3,6 | 96,1 | 0,4 | 100 |
| 9 | mit Katalysator | 200 | 0,052 | 60 | 2 | n. b. | n. b. | n. b. |
| 10 | mit Katalysator | | | | | | | |
| | mit 5 Ma.-% H₂O bezogen auf oAB | 200 | 0,030 | 60 | 1,4 | 98 | 0,6 | >99 |
| 11 | mit Katalysator | 200 | 0,041 | 60 | 0,9 | 99 | 0,1 | 100 |
| | mit 30 Ma.-% H₂O bezogen auf oAB | | | | | | | |

| | | | | | | | | |
|---|---|---|---|---|---|---|---|---|
| Erläuterung der Abkürzungen: n. b. = nicht bestimmt, ΣA% = Summe der Flächenprozente (der drei Komponenten oAB, ANL und Amid) | | | | | | | | |

## Patentansprüche

1. Verfahren zur Herstellung von Anilin oder eines Anilinfolgeprodukts, umfassend die Schritte:
(I) Bereitstellen einer Lösung von Aminobenzoesäure in Anilin, wobei ein auf die Gesamtmasse aus Aminobenzoesäure und Anilin bezogener Massenanteil an Anilin in der Lösung im Bereich von 20 % bis 85 % eingestellt wird;
(II) Umsetzen von in der in Schritt (I) bereitgestellten Lösung enthaltenden Aminobenzoesäure zu Anilin in einem Reaktor durch thermisches Decarboxylieren bei einer Temperatur im Bereich von 165 °C bis 500 °C ohne die Anwesenheit eines systemfremden Katalysators;
(III) optional, Umsetzen von in Schritt (II) erhaltenem Anilin zu einem Anilinfolgeprodukt.

2. Verfahren gemäß Anspruch 1, bei welchem zur Durchführung von Schritt (I) Aminobenzoesäure und Anilin in einem diskontinuierlich oder kontinuierlich betriebenen Mischer vermischt werden.

3. Verfahren gemäß Anspruch 2, bei welchem der Mischer aus Schritt (I) und der Reaktor aus Schritt (II) kontinuierlich betrieben werden.

4. Verfahren gemäß Anspruch 2, bei welchem zur Durchführung von Schritt (I) mehrere, insbesondere zwei, parallel geschaltete, diskontinuierlich betriebene Mischer eingesetzt werden und der Reaktor aus Schritt (II) kontinuierlich betrieben wird, wobei zu jedem Zeitpunkt des kontinuierlichen Betriebs des Reaktors aus Schritt (II) aus einem der in Schritt (I) eingesetzten Mischer die Lösung der Aminobenzoesäure in den Reaktor aus Schritt (II) eingetragen wird, während in einem anderen dieser Mischer das Vermischen der Aminobenzoesäure in Anilin abläuft.

5. Verfahren gemäß einem der vorstehenden Ansprüche, bei welchem das thermische Decarboxylieren der Aminobenzoesäure ein erster Teilschritt (II)(1) des Schrittes (II) ist, an welchen sich ein zweiter Teilschritt (II)(2) anschließt, in dem in Teilschritt (II)(1) gebildetes Anilin gereinigt wird.

6. Verfahren gemäß Anspruch 5, bei welchem das in Schritt (I) eingesetzte Anilin
dem in Teilschritt (II)(1) gebildeten Anilin,
dem in Teilschritt (II)(2) gereinigten Anilin
oder
dem in Teilschritt (II)(1) gebildeten Anilin und dem in Teilschritt (II)(2) gereinigten Anilin
entnommen wird.

7. Verfahren gemäß einem der vorstehenden Ansprüche, bei welchem in Schritt (I) Aminobenzoesäure bei einer Temperatur im Bereich von -6 °C bis 120 °C in Anilin gelöst wird.

8. Verfahren gemäß Anspruch 7, bei welchem Aminobenzoesäure und Anilin zunächst bei einer Temperatur im Bereich von -6 °C bis 100 °C vermischt und anschließend in einer Inertgasatmosphäre auf eine Temperatur im Bereich von > 100 °C bis 120 °C erhitzt werden.

9. Verfahren gemäß einem der vorstehenden Ansprüche, bei welchem das thermische Decarboxylieren in Schritt (II) bei einem Druck im Bereich von 4,0 bar_{(abs.)} bis 30 bar_{(abs.)} durchgeführt wird.

10. Verfahren gemäß einem der Ansprüche 5 bis 9, bei welchem dem Reaktor aus Schritt (II) kontinuierlich ein flüssiger, Anilin enthaltender Strom und ein gasförmiger, Kohlenstoffdioxid und gasförmiges Anilin enthaltender Strom entnommen werden, wobei der gasförmige Strom einen Kondensator durchläuft, in dem gasförmiges Anilin verflüssigt wird und aus dem Kohlenstoffdioxid gasförmig ausgetragen wird, wobei in dem Kondensator erhaltenes flüssiges Anilin dem Teilschritt (II)(1) und/oder dem Teilschritt (II)(2) zugeführt wird.

11. Verfahren gemäß einem der vorstehenden Ansprüche, bei welchem das Bereitstellen der Lösung von Aminobenzoesäure in Anilin in Schritt (I) die chemische Herstellung von Aminobenzoesäure umfasst.

12. Verfahren gemäß einem der Ansprüche 1 bis 10, bei welchem das Bereitstellen der Lösung von Aminobenzoesäure in Anilin in Schritt (I) folgende Teilschritte umfasst:
(I)(1) Fermentieren eines Rohstoffes, der wenigstens
• eine fermentierbare Kohlenstoff-haltige Verbindung und
• eine Stickstoff-haltige Verbindung
umfasst,
in einem Fermentationsreaktor unter Verwendung von Mikroorganismen unter Erhalt einer Aminobenzoat und/oder Aminobenzoesäure enthaltenden Fermentationsbrühe,
Gewinnen von Aminobenzoesäure aus der Fermentationsbrühe;
und
(I)(2) Lösen der in Schritt (I)(1) aus der Fermentationsbrühe gewonnenen Aminobenzoesäure in Anilin.

13. Verfahren gemäß Anspruch 12, bei welchem die in Schritt (I)(1) eingesetzten Mikroorganismen eine Art ausgewählt aus der Gruppe bestehend aus *Escherichia coli, Pseudomonas putida, Corynebacterium glutamicum, Ashbya gossypii, Pichia pastoris, Hansenula polymorpha, Kluyveromyces marxianus, Yarrowia lipolytica, Zygosaccharomyces bailii* und *Saccharomyces cerevisiae,* umfassen.

14. Verfahren gemäß einem der vorstehenden Ansprüche, bei welchem die zum Bereitstellen der Lösung von Aminobenzoesäure in Anilin in Schritt (I) eingesetzte Aminobenzoesäure Wasser in einem auf die Gesamtmasse aus Aminobenzoesäure und Wasser bezogenen Massenanteil im Bereich von 0,1 % bis 40 % umfasst.

15. Verfahren gemäß einem der vorstehenden Ansprüche, umfassend Schritt (III), wobei Schritt (III) eine der folgenden Umsetzungen umfasst:
(III)(1) säurekatalysierte Reaktion des Anilins mit Formaldehyd unter Bildung von Di- und Polyaminen der Diphenylmethanreihe;
(III)(2) säurekatalysierte Reaktion des Anilins mit Formaldehyd, gefolgt von Umsetzung mit Phosgen unter Bildung von Di- und Polyisocyanaten der Diphenylmethanreihe;
(III)(3) Umsetzung des Anilins zu einer Azoverbindung.

## Claims

1. Process for preparing aniline or an aniline conversion product, comprising the steps of:
(I) providing a solution of aminobenzoic acid in aniline, with establishment of a proportion by mass of aniline in the solution based on the total mass of aminobenzoic acid and aniline in the range from 20% to 85%;
(II) converting aminobenzoic acid containing in the solution provided in step (I) to aniline in a reactor by thermally decarboxylating at a temperature in the range from 165°C to 500°C without the presence of any catalyst extraneous to the system;
(III) optionally converting aniline obtained in step (II) to an aniline conversion product.

2. Process according to Claim 1, in which step (I) is performed by mixing aminobenzoic acid and aniline in a batchwise or continuous mixer.

3. Process according to Claim 2, in which the mixer from step (I) and the reactor from step (II) are operated continuously.

4. Process according to Claim 2, in which step (I) is performed using multiple, especially two, batchwise mixers connected in parallel and the reactor from step (II) is operated continuously, wherein, at any time in the continuous operation of the reactor from step (II), the solution of the aminobenzoic acid from one of the mixers used in step (I) is introduced into the reactor from step (II), while the mixing of the aminobenzoic acid into aniline is proceeding in another of these mixers.

5. Process according to any of the preceding claims, in which the thermal decarboxylating of the aminobenzoic acid is a first partial step (II)(1) of step (II), which is followed by a second partial step (II)(2) in which aniline formed in partial step (II)(1) is purified.

6. Process according to Claim 5, in which the aniline used in step (I) is taken
from the aniline formed in partial step (II)(1), from the aniline purified in partial step (II)(2) or
from the aniline formed in partial step (II)(1) and from the aniline purified in partial step (II) (2) .

7. Process according to any of the preceding claims, in which, in step (I), aminobenzoic acid is dissolved in aniline at a temperature in the range from -6°C to 120°C.

8. Process according to Claim 7, in which aminobenzoic acid and aniline are first mixed at a temperature in the range from -6°C to 100°C and then heated in an inert gas atmosphere to a temperature in the range from > 100°C to 120°C.

9. Process according to any of the preceding claims, in which the thermal decarboxylating in step (II) is performed at a pressure in the range from 4.0 bar_{(abs.)} to 30 bar_{(abs.)}.

10. Process according to any of Claims 5 to 9, in which a liquid, aniline-containing stream and a gaseous, carbon dioxide- and gaseous aniline-containing stream are taken continuously from the reactor from step (II), wherein the gaseous stream passes through a condenser in which gaseous aniline is liquefied and from which carbon dioxide is discharged in gaseous form, wherein liquid aniline obtained in the condenser is fed to partial step (II)(1) and/or to partial step (II)(2).

11. Process according to any of the preceding claims, in which the providing of the solution of aminobenzoic acid in aniline in step (I) comprises the chemical preparation of aminobenzoic acid.

12. Process according to any of Claims 1 to 10, in which the providing of the solution of aminobenzoic acid in aniline in step (I) comprises the following partial steps:
(I) (1) fermenting a raw material comprising at least
□ a fermentable carbon-containing compound and
□ a nitrogen-containing compound,
in a fermentation reactor using microorganisms to obtain an aminobenzoate- and/or aminobenzoic acid-containing fermentation broth,
obtaining aminobenzoic acid from the fermentation broth;
and
(I)(2) dissolving the aminobenzoic acid obtained from the fermentation broth in step (I)(1) in aniline.

13. Process according to Claim 12, in which the microorganisms used in step (I)(1) comprise a species selected from the group consisting of *Escherichia coli, Pseudomonas putida, Corynebacterium glutamicum, Ashbya gossypii, Pichia pastoris, Hansenula polymorpha, Kluyveromyces marxianus, Yarrowia lipolytica, Zygosaccharomyces bailii* and *Saccharomyces cerevisiae.*

14. Process according to any of the preceding claims, in which the aminobenzoic acid used to provide the solution of aminobenzoic acid in aniline in step (I) comprises water in a proportion by mass based on the total mass of aminobenzoic acid and water in the range from 0.1% to 40%.

15. Process according to any of the preceding claims, comprising step (III), wherein step (III) comprises one of the following conversions:
(III) (1) acid-catalysed reaction of aniline with formaldehyde to form di- and polyamines of the diphenylmethane series;
(III) (2) acid-catalysed reaction of aniline with formaldehyde, followed by reaction with phosgene to form di- and polyisocyanates of the diphenylmethane series;
(III)(3) conversion of aniline to an azo compound.

## Revendications

1. Procédé de préparation d'aniline ou d'un produit dérivé d'aniline, comprenant les étapes :
(I) mise à disposition d'une solution d'acide aminobenzoïque dans de l'aniline, une proportion en masse d'aniline dans la solution, par rapport à la masse totale d'acide aminobenzoïque et d'aniline, étant ajustée dans la plage de 20 % à 85 % ;
(II) transformation de l'acide aminobenzoïque contenant la solution mise à disposition dans l'étape (I) en aniline dans un réacteur par décarboxylation thermique à une température dans la plage de 165 °C à 500 °C en l'absence d'un catalyseur externe au système ;
(III) éventuellement, transformation de l'aniline obtenue dans l'étape (II) en un produit dérivé d'aniline.

2. Procédé selon la revendication 1, dans lequel pour la mise en œuvre de l'étape (I), l'acide aminobenzoïque et l'aniline sont mélangés dans un mélangeur exploité de manière discontinue ou continue.

3. Procédé selon la revendication 2, dans lequel le mélangeur de l'étape (I) et le réacteur de l'étape (II) sont exploités de manière continue.

4. Procédé selon la revendication 2, dans lequel pour la mise en œuvre de l'étape (I), plusieurs, en particulier deux mélangeurs montés en parallèle, exploités de manière discontinue sont utilisés et le réacteur de l'étape (II) est exploité de manière continue, dans lequel à chaque instant de l'exploitation de manière continue du réacteur de l'étape (II), la solution de l'acide aminobenzoïque, depuis un des mélangeurs utilisés dans l'étape (I), est introduite dans le réacteur de l'étape (II), tandis que dans un autre de ces mélangeurs, le mélange de l'acide aminobenzoïque dans l'aniline a lieu.

5. Procédé selon l'une quelconque des revendications précédentes, dans lequel la décarboxylation thermique de l'acide aminobenzoïque est une première étape partielle (II)(1) de l'étape (II), qui est suivie par une deuxième étape partielle (II)(2), dans laquelle l'aniline formée dans l'étape partielle (II)(1) est purifiée.

6. Procédé selon la revendication 5, dans lequel l'aniline utilisée dans l'étape (I) est prélevée de l'aniline formée dans l'étape partielle (II)(1), de l'aniline formée dans l'étape partielle (II)(2), ou de l'aniline formée dans l'étape partielle (II)(1) et de l'aniline purifiée dans l'étape partielle (II)(2).

7. Procédé selon l'une quelconque des revendications précédentes, dans lequel dans l'étape (I) l'acide aminobenzoïque est dissous dans l'aniline à une température dans la plage de -6 °C à 120 °C.

8. Procédé selon la revendication 7, dans lequel l'acide aminobenzoïque et l'aniline sont mélangés tout d'abord à une température dans la plage de -6 °C à 100 °C et ensuite sont chauffés dans une atmosphère de gaz inerte à une température dans la plage de > 100 °C à 120 °C.

9. Procédé selon l'une quelconque des revendications précédentes, dans lequel la décarboxylation thermique dans l'étape (II) est réalisée à une pression dans la plage de 4,0 bars_{(abs.)} à 30 bars_{(abs.)}.

10. Procédé selon l'une quelconque des revendications 5 à 9, dans lequel un flux liquide contenant de l'aniline et un flux gazeux contenant du dioxyde de carbone et de l'aniline gazeuse sont prélevés de manière continue du réacteur de l'étape (II), le flux gazeux traversant un condenseur dans lequel de l'aniline gazeuse est liquéfiée et duquel du dioxyde de carbone est évacué sous forme gazeuse, de l'aniline liquide obtenue dans le condenseur étant alimentée à l'étape partielle (II)(1) et/ou à l'étape partielle (II)(2).

11. Procédé selon l'une quelconque des revendications précédentes, dans lequel la mise à disposition de la solution d'acide aminobenzoïque dans l'aniline dans l'étape (I) comprend la préparation chimique de l'acide aminobenzoïque.

12. Procédé selon l'une quelconque des revendications 1 à 10, dans lequel la mise à disposition de la solution d'acide aminobenzoïque dans l'aniline dans l'étape (I) comprend les étapes partielles suivantes
(I)(1) fermentation d'une matière première qui comprend au moins
• un composé pouvant être fermenté contenant du carbone et
• un composé contenant de l'azote,
dans un réacteur de fermentation en utilisant des micro-organismes avec obtention d'un bouillon de fermentation contenant un aminobenzoate et/ou de l'acide aminobenzoïque,
obtention d'acide aminobenzoïque à partir du bouillon de fermentation ;
et
(I)(2) dissolution de l'acide aminobenzoïque obtenu depuis le bouillon de fermentation dans l'étape (I)(1) dans l'aniline.

13. Procédé selon la revendication 12, dans lequel les micro-organismes utilisés dans l'étape (I)(1) comprennent une sorte choisie dans le groupe constitué par *Escherichia coli, Pseudomonas putida, Corynebacterium glutamicum, Ashbya gossypii, Pichia pastoris, Hansenula polymorpha, Kluyveromyces marxianus, Yarrowia lipotytica, Zygosaccharomyces bailii* et *Saccharomyces cerevisiae.*

14. Procédé selon l'une quelconque des revendications précédentes, dans lequel l'acide aminobenzoïque utilisé dans l'étape (I) pour la mise à disposition de la solution d'acide aminobenzoïque dans l'aniline comprend de l'eau en une proportion en masse, par rapport à la masse totale d'acide aminobenzoïque et d'eau, dans la plage de 0,1 % à 40 %.

15. Procédé selon l'une quelconque des revendications précédentes, comprenant l'étape (III), l'étape (III) comprenant l'une des transformations suivantes :
(III) (1) réaction catalysée par un acide de l'aniline avec du formaldéhyde avec formation de diamines et de polyamines de la série du diphénylméthane ;
(III) (2) réaction catalysée par un acide de l'aniline avec du formaldéhyde, suivie par une transformation avec du phosgène avec formation de diisocyanates et de polyisocyanates de la série du diphénylméthane ;
(III)(3) transformation de l'aniline en un composé de type azo.
